(19) Europäisches Patentamt
European Patent Office
Office européen des brevets

(11) **EP 3 296 325 A1**

(12) **EUROPEAN PATENT APPLICATION**
published in accordance with Art. 153(4) EPC

(43) Date of publication:
**21.03.2018 Bulletin 2018/12**

(51) Int Cl.:
**C08F 2/04** (2006.01)

(21) Application number: **16792601.3**

(22) Date of filing: **02.05.2016**

(86) International application number:
**PCT/JP2016/063543**

(87) International publication number:
**WO 2016/181876 (17.11.2016 Gazette 2016/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.05.2015 JP 2015095685**

(71) Applicant: **Nisshinbo Holdings Inc.**
**Tokyo 103-8650 (JP)**

(72) Inventors:
• **HAYAKAWA Kazutoshi**
**Chiba-shi**
**Chiba 267-0056 (JP)**
• **HASHIBA Toshifumi**
**Chiba-shi**
**Chiba 267-0056 (JP)**
• **MATSUZAKA Erina**
**Chiba-shi**
**Chiba 267-0056 (JP)**

(74) Representative: **Bailey, Sam Rogerson et al**
**Mewburn Ellis LLP**
**City Tower**
**40 Basinghall Street**
**London EC2V 5DE (GB)**

(54) **ELLIPTICAL, NEEDLE-SHAPED, OR ROD-SHAPED CROSSLINKED POLYMER PARTICLES, AND USE THEREOF**

(57) Provided are elliptical, needle-shaped, or rod-shaped crosslinked polymer particles in which 5-100 mol% of all repeating units are repeating units derived from an unsaturated monomer having a functional group selected from an epoxy group, a carboxyl group, an amide group, a hydroxy group, an amino group, and a thiol group, wherein (1) the average ($L_{AV}$) length (L) in a two-dimensional projection diagram obtained by irradiating the particles with light from a direction orthogonal to the longitudinal direction is 0.1-80 $\mu$m, (2) the average ($D_{AV}$) breadth (D) in the two-dimensional projection diagram obtained by irradiating the particles with light from the direction orthogonal to the longitudinal direction is 0.05-40 $\mu$m, and (3) the average ($P_{AV}$) aspect ratio (L/D) calculated from the length (L) and the breadth (D) is 1.5-30.

**Description**

TECHNICAL FIELD

The present invention relates to elliptical, needle-shaped or rod-shaped crosslinked polymer particles, and to uses thereof.

BACKGROUND ART

Micron-size polymer particles and inorganic particles are used as fillers and specimens in a variety of fields, such as electrical and electronic materials, optical materials, paints, inks, construction materials, biological and pharmaceutical materials, and cosmetics. In recent years, active research has been carried out particularly on particles of unusual, non-spherical shapes. Because such particles confer diverse properties, including optical characteristics and tactile feel, new applications are constantly being developed.

The inventors have been working on the development of elliptical or needle-shaped polymer particles of high aspect ratio, and have discovered particles with characteristics superior to those of conventional spherical particles in terms of such properties as hiding power, light-diffusing ability and tactile qualities (Patent Documents 1 and 2).

However, in many applications where organic solvents and heat (e.g., molded articles) are applied, both the heat resistance and the chemical resistance have remained inadequate. Also, when pressure is applied, the particles bend easily and the properties imparted by the elliptical shape are lost.

Crosslinking of the polymer is carried out so as to increase the heat resistance and chemical resistance of polymer particles. Crosslinked polymer particles can generally be obtained by adding, within the monomer, a suitable amount of a polyfunctional unsaturated monomer. However, although this approach is excellent for obtaining spherical particles, efficiently obtaining elliptical crosslinked polymer particles is difficult. In investigations by the inventors, when attempts were made to obtain crosslinked particles by adding such a polyfunctional unsaturated monomer, agglomerates increased or it became impossible to maintain an elliptical shape, with a spherical or approximately spherical shape often resulting instead; efficiently obtaining good elliptical crosslinked polymer particles was difficult. Also, because the speed at which the crosslinking reaction proceeds becomes more rapid as the amount of the crosslinking ingredient increases, it is quite difficult to stably obtain particles having a high aspect ratio (length/breadth).

In light of such circumstances, there has existed a desire for crosslinked polymer particles that can be used in applications requiring heat resistance, chemical resistance, and resistance to hot chemicals.

PRIOR ART DOCUMENTS

PATENT DOCUMENTS

Patent Document 1: JP-A 2009-235353
Patent Document 2: JP-A 2009-235355

SUMMARY OF THE INVENTION

PROBLEMS TO BE SOLVED BY THE INVENTION

It is therefore an object of the present invention to provide elliptical, needle-shaped or rod-shaped crosslinked polymer particles which, while retaining such properties as a light-diffusing ability, UV-cutting properties and tactile qualities, also have an excellent heat resistance, chemical resistance and resistance to hot chemicals. Another object of the invention is to provide uses for such particles.

MEANS FOR SOLVING THE PROBLEMS

The inventors have conducted extensive investigations aimed at achieving the above objects, discovering as a result that elliptical, needle-shaped or rod-shaped crosslinked polymer particles obtained by solution polymerization using an unsaturated monomer having a specific functional group possess an improved heat resistance, chemical resistance and resistance to hot chemicals.

Accordingly, the invention provides the following elliptical, needle-shaped or rod-shaped crosslinked polymer particle, and uses thereof.

1. An elliptical, needle-shaped or rod-shaped crosslinked polymer particle including, as 5 to 100 mol% of all recurring units, recurring units derived from an unsaturated monomer having a functional group selected from epoxy, carboxyl, amide, hydroxyl, amino and thiol groups, wherein

(1) a two-dimensional projection obtained by irradiating the particle with light from a direction orthogonal to a long axis of the particle has a length L with an average value $L_{AV}$ of from 0.1 to 80 μm,
(2) a two-dimensional projection obtained by irradiating the particle with light from a direction orthogonal to a long axis of the particle has a breadth D with an average value $D_{AV}$ of from 0.05 to 40 μm, and
(3) the aspect ratio L/D calculated from the length L and breadth D has an average value $P_{AV}$ of from 1.5 to 30.

2. The elliptical, needle-shaped or rod-shaped crosslinked polymer particle of 1 above, wherein the functional group is an epoxy, carboxyl, amino or amide group.

3. The elliptical, needle-shaped or rod-shaped crosslinked polymer particle of 1 or 2 above, wherein the crosslinked polymer further includes recurring units derived from at least one type of unsaturated monomer selected from styrenic monomers, (meth)acrylic ester monomers and vinyl carboxylate monomers.

4. The elliptical, needle-shaped or rod-shaped crosslinked polymer particle of any of 1 to 3 above, wherein the ratio SB/SD between the actual specific surface area SB of the particle and the theoretical specific surface area SD of a spherical particle calculated from the volume mean particle size of the elliptical, needle-shaped or rod-shaped crosslinked polymer particle satisfies the condition SB/SD ≥ 1.2.

5. The elliptical, needle-shaped or rod-shaped crosslinked polymer particle of any of 1 to 4 above wherein, when the crosslinked polymer particles are added to at least one solvent selected from the group consisting of ethanol, toluene, ethyl acetate, methyl ethyl ketone, dimethylformamide and dipropylene glycol and stirred for 30 minutes at 27°C, the percent loss in weight is not more than 10 wt%.

6. The elliptical, needle-shaped or rod-shaped crosslinked polymer particle of any of 1 to 5 above wherein, when the crosslinked polymer particles are added to at least one solvent selected from the group consisting of ethanol, toluene, ethyl acetate, methyl ethyl ketone, dimethylformamide and dipropylene glycol and stirred for 30 minutes at 70°C, the percent loss in weight is not more than 10 wt%.

7. A resin composition obtained using the elliptical, needle-shaped or rod-shaped crosslinked polymer particle of any of 1 to 6 above.

8. A light-diffusing sheet obtained using the elliptical, needle-shaped or rod-shaped crosslinked polymer particle of any of 1 to 6 above.

9. A paint composition obtained using the elliptical, needle-shaped or rod-shaped crosslinked polymer particle of any of 1 to 6 above.

10. An ink composition obtained using the elliptical, needle-shaped or rod-shaped crosslinked polymer particle of any of 1 to 6 above.

11. A cosmetic preparation obtained using the elliptical, needle-shaped or rod-shaped crosslinked polymer particle of any of 1 to 6 above.

12. A material for the electrical or electronics industry obtained using the elliptical, needle-shaped or rod-shaped crosslinked polymer particle of any of 1 to 6 above.

13. An adhesive obtained using the elliptical, needle-shaped or rod-shaped crosslinked polymer particle of any of 1 to 6 above.

14. A thermally cavitated product having pores obtained using the elliptical, needle-shaped or rod-shaped crosslinked polymer particle of any of 1 to 6 above.

15. A diagnostic agent for medical use obtained using the elliptical, needle-shaped or rod-shaped crosslinked polymer particle of any of 1 to 6 above.

16. A method for producing the elliptical, needle-shaped or rod-shaped crosslinked polymer particle of any of 1 to 6 above, comprising the step of carrying out solution polymerization by heating a synthesis solution containing a mixed solvent of water, a hydrophilic organic solvent and a hydrophobic organic solvent, a high-molecular-weight stabilizer, a polymerization initiator and the unsaturated monomer; and, at least following the start of heating, adjusting the pH of the synthesis solution to 5 or less or to 9 or more.

17. The method for producing the elliptical, needle-shaped or rod-shaped crosslinked polymer particle of 16 above, wherein the mixing ratio of the water, hydrophilic organic solvent and hydrophobic organic solvent, expressed as a weight ratio, is from 99:0.5:0.5 to 25:55:20.

18. The method for producing the elliptical, needle-shaped or rod-shaped crosslinked polymer particle of 16 or 17 above, wherein the hydrophobic organic solvent is an organic compound having a molecular weight of at least 200.

ADVANTAGEOUS EFFECTS OF THE INVENTION

The elliptical, needle-shaped or rod-shaped crosslinked polymer particle of the invention, while retaining properties distinctive to elliptical polymer particles, such as light-diffusing ability, UV-cutting properties and tactile qualities, also has an excellent heat resistance, chemical resistance and resistance to hot chemicals.

BRIEF DESCRIPTION OF THE DIAGRAMS

[FIG. 1] FIG. 1 shows a scanning electron micrograph of particles obtained in Synthesis Example 1.
[FIG. 2] FIG. 2 is a diagram showing the light scattering distribution of reflected light obtained using an automated goniophotometer in test sheets prepared in Working Example 9 and Comparative Example 8.

EMBODIMENT FOR CARRYING OUT THE INVENTION

[Elliptical, Needle-Shaped or Rod-Shaped Crosslinked Polymer Particle]

The elliptical, needle-shaped or rod-shaped crosslinked polymer particle of the invention (also referred to below simply as "the crosslinked polymer particle") is a polymer particle which includes, as 5 to 100 mol% of all recurring units, recurring units derived from an unsaturated monomer (referred to below as "Unsaturated Monomer A") having a functional group selected from epoxy, carboxyl, amide, hydroxyl, amino and thiol groups, and is characterized in that:

(1) a two-dimensional projection obtained by irradiating the particle with light from a direction orthogonal to a long axis of the particle has a length L with an average value $L_{AV}$ of from 0.1 to 80 $\mu$m;
(2) a two-dimensional projection obtained by irradiating the particle with light from a direction orthogonal to a long axis of the particle has a breadth D with an average value $D_{AV}$ of from 0.05 to 40 $\mu$m; and
(3) the aspect ratio L/D calculated from the length L and breadth D has an average value $P_{AV}$ of from 1.5 to 30.

The $L_{AV}$ value for the crosslinked polymer particle is from 0.1 to 80 $\mu$m, preferably from 0.2 to 60 $\mu$m, more preferably from 1.0 to 40 $\mu$m, and even more preferably from 2 to 30 $\mu$m. When $L_{AV}$ is greater than 80 $\mu$m, the properties become no different from those of widely used fibers and so the particle loses its superiority. Also, the decrease in specific surface area per unit tends to be accompanied by a marked decrease in optical characteristics such as the light scattering properties. On the other hand, when $L_{AV}$ is less than 0.1 $\mu$m, the breadth of the particle also becomes small, which may lead to a decline in the optical characteristics and a decrease in strength.
The $D_{AV}$ value for the crosslinked polymer particle is from 0.05 to 40 $\mu$m, preferably from 0.1 to 30 $\mu$m, more preferably from 0.5 to 20 $\mu$m, and even more preferably from 1 to 15 $\mu$m. When $D_{AV}$ is greater than 40 $\mu$m, the properties become no different from those of widely used spherical particles. Also, the decrease in specific surface area per unit tends to be accompanied by a marked decrease in optical characteristic effects. On the other hand, when $D_{AV}$ is less than 0.05 $\mu$m, the optical characteristics may decline and the strength may decrease.
The $P_{AV}$ value for the crosslinked polymer particle is from 1.5 to 30, preferably from 1.8 to 25, more preferably from 2 to 20, and even more preferably from 2.2 to 20. In cases where optical characteristics such as the light scattering properties are of particular importance, it is best for $P_{AV}$ to be from 3 to 18. When $P_{AV}$ is greater than 30 $\mu$m, the resulting particles tend to orient themselves, as a result of which optical characteristics such as light scattering properties and light reflectivity cannot be stably obtained. On the other hand, when $P_{AV}$ is less than 1.5, only optical characteristic effects of the same degree as those by spherical particles made of the same ingredients are obtained, and so the crosslinked polymer particle provides little advantage.
Also, the crosslinked polymer particle has a volume mean particle size (MV) which is preferably from 0.06 to 50 $\mu$m, more preferably from 0.1 to 30 $\mu$m, and even more preferably from 0.5 to 20 $\mu$m. When MV is greater than 50 $\mu$m, the decrease in specific surface area per unit may be accompanied by a decrease in the optical characteristic effects. On the other hand, when MV is less than 0.06 $\mu$m, light may pass through, reducing the optical characteristic effects.
In this invention, "volume mean particle size" is a measured value obtained by the laser scattering diffraction method and refers to, in the case of elliptical, needle-shaped or rod-shaped particles or unusually shaped particles, the mean diameter of spheres having the same volumes as the particles (i.e., mean sphere-equivalent diameter of the particles).
Unsaturated Monomer A contains a functional group selected from epoxy, carboxyl, amide, hydroxyl, amino and thiol groups. Unsaturated Monomer A is exemplified as shown below. In the explanation that follows, "$C_n$" means

"n number of" carbon atoms.

(1) Epoxy Group-Containing Unsaturated Monomers

Examples include epoxy group-containing (meth)acrylates such as glycidyl (meth)acrylate, (β-methyl)glycidyl (meth)acrylate, 3,4-epoxycyclohexyl (meth)acrylate, glycidyl α-ethylacrylate, glycidyl α-n-propylacrylate, glycidyl α-n-butylacrylate, 3,4-epoxybutyl acrylate, 3,4-epoxybutyl methacrylate, 4,5-epoxypentyl methacrylate, 6,7-epoxyheptyl acrylate, 6,7-epoxyheptyl methacrylate and 6,7-epoxyheptyl α-ethylacrylate; vinyl glycidyl ethers such as o-vinylphenyl glycidyl ether, m-vinylphenyl glycidyl ether, p-vinylphenyl glycidyl ether, o-vinylbenzyl glycidyl ether, m-vinylbenzyl glycidyl ether and p-vinylbenzyl glycidyl ether; and monomers having an ethylenically unsaturated bond and an epoxy group, such as 2,3-diglycidyloxystyrene, 3,4-diglycidyloxystyrene, 2,4-diglycidyloxystyrene, 3,5-diglycidyloxystyrene, 2,6-diglycidyloxystyrene, 5-vinylpyrogallol triglycidyl ether, 4-vinylpyrogallol triglycidyl ether, vinylfluoroglycinol triglycidyl ether, 2,3-dihydroxymethylstyrene diglycidyl ether, 3,4-dihydroxymethylstyrene diglycidyl ether, 2,4-dihydroxymethylstyrene diglycidyl ether, 3,5-dihydroxymethylstyrene diglycidyl ether, 2,6-dihydroxymethylstyrene diglycidyl ether, 2,3,4-trihydroxymethylstyrene triglycidyl ether, 1,3,5-trihydroxymethylstyrene triglycidyl ether, allyl glycidyl ether, 3,4-epoxyvinylcyclohexane, di(β-methyl)glycidyl maleate and di(β-methyl)glycidyl fumarate.

(2) Carboxyl Group-Containing Unsaturated Monomers

Examples include unsaturated carboxylic acids such as (meth)acrylic acid, crotonic acid, cinnamic acid, itaconic acid, maleic acid and fumaric acid; mono($C_{1-8}$ alkyl) esters of itaconic acid such as monobutyl itaconate; mono($C_{1-8}$ alkyl) esters of maleic acid such as monobutyl maleate; vinyl group-containing aromatic carboxylic acids such as vinylbenzoic acid; and salts thereof.

(3) Amide Group-Containing Unsaturated Monomers

Examples include (meth)acrylamide, α-ethyl (meth)acrylamide, N-methyl (meth)acrylamide, N-butoxymethyl (meth)acrylamide, diacetone (meth)acrylamide, N,N-dimethyl (meth)acrylamide, N,N-diethyl (meth)acrylamide, N,N-dimethyl-p-styrene sulfonamide, N,N-dimethylaminoethyl (meth)acrylamide, N,N-diethylaminoethyl (meth)acrylamide, N,N-dimethylaminopropyl (meth)acrylamide and N,N-diethylaminopropyl (meth)acrylamide.

(4) Hydroxyl Group-Containing Unsaturated Monomers

Examples include hydroxyl group-containing (meth)acrylic monomers such as 2-hydroxyethyl (meth)acrylate, 2-hydroxypropyl (meth)acrylate, 3-hydroxypropyl (meth)acrylate and 4-hydroxybutyl (meth)acrylate; polyalkylene glycol (meth)acrylic monomers such as polyethylene glycol mono(meth)acrylate and polypropylene glycol mono(meth)acrylate; hydroxyalkyl vinyl ether monomers such as hydroxyethyl vinyl ether and hydroxybutyl vinyl ether; and hydroxyl group-containing allyl monomers such as allyl alcohol and 2-hydroxyethyl allyl ether.

(5) Amino Group-Containing Unsaturated Monomers

Examples include allylamine monomers such as allylamine and N-methylallylamine; amino group-containing styrenic monomers such as p-aminostyrene; amino group-containing acrylic monomers such as 2-aminoethyl (meth)acrylate and 2-(dimethylamino)ethyl methacrylate; and triazine-containing monomers such as 2-vinyl-4,6-diamino-S-triazine. Of these, compounds having a primary or secondary amino group are preferred.

(6) Thiol (Mercapto) Group-Containing Unsaturated Monomers

Examples include mercapto group-containing (meth)acrylic monomers such as N-(2-mercaptoethyl) acrylamide, N-(2-mercapto-1-carboxyethyl) acrylamide, N-(2-mercaptoethyl) methacrylamide, N-(4-mercaptophenyl) acrylamide, N-(7-mercaptonaphthyl) acrylamide, maleic acid mono(2-mercaptoethylamide), 2-mercaptoethyl (meth)acrylate and 2-mercapto-1-carboxyethyl (meth)acrylate.

Unsaturated Monomer A may be a monomer that includes one of the above functional groups or may be a monomer that includes two or more. Unsaturated Monomer A may be of one type used alone or of two or more types used in combination.

In terms of being able to stably and efficiently obtain crosslinked particles in a relatively low temperature region and also from industrial, practical and cost standpoints, preferred examples of Unsaturated Monomer A include epoxy group-containing monomers such as glycidyl (meth)acrylate, (β-methyl)glycidyl (meth)acrylate, 3,4-epoxycyclohexyl (meth)acrylate, allyl glycidyl ether, 3,4-epoxyvinylcyclohexane, di(β-methyl)glycidyl maleate and di(β-methyl)glycidyl fumarate; carboxyl group-containing monomers such as (meth)acrylic acid; amide group-containing monomers such as (meth)acrylamide, N-methyl (meth)acrylamide, N,N-dimethyl (meth)acrylamide and N,N-diethyl (meth)acrylamide; hydroxyl group-containing monomers, including hydroxyalkyl (meth)acrylates such as 2-hydroxyethyl (meth)acrylate, 2-hydroxypropyl (meth)acrylate, 3-hydroxypropyl (meth)acrylate and 4-hydroxybutyl (meth)acrylate, and polyalkylene glycol (meth)acrylates such as polyethylene glycol (meth)acrylate and polypropylene glycol (meth)acrylate; and amino group-containing monomers such as p-aminostyrene and 2-aminoethyl (meth)acrylate. Of these, epoxy group-containing unsaturated monomers, carboxyl group-containing unsaturated monomers, amide group-containing unsaturated monomers and amino group-containing monomers are more preferred; epoxy group-containing unsaturated monomers, carboxyl group-containing unsaturated monomers and amide group-containing unsaturated monomers are even more preferred; and epoxy group-containing monomers are most preferred.

In the polymer particle of the invention, from 5 to 100 mol%, preferably from 6 to 80 mol%, more preferably from 7 to 50 mol%, and even more preferably from 8 to 40 mol%, of all recurring units are recurring units derived from Unsaturated Monomer A. At a content of recurring units derived from Unsaturated Monomer A in this range, crosslinking reactions derived from a functional group selected from epoxy, carboxyl, amide, hydroxyl, amino and thiol groups included in Unsaturated Monomer A proceed efficiently, enabling the resulting crosslinked polymer particle to be imparted with sufficient heat resistance and chemical resistance.

The crosslinked polymer particle of the invention may be one that includes only recurring units derived from Unsaturated Monomer A, but may additionally include recurring units derived from an unsaturated monomer other than Unsaturated Monomer A (referred to below as "Unsaturated Monomer B").

Unsaturated Monomer B is exemplified by styrenic monomers, (meth)acrylic ester monomers, vinyl carboxylate monomers, N-vinyl compound monomers, olefinic monomers, fluorinated olefinic monomers, conjugated diene monomers and ionic functional group-containing monomers.

Examples of styrenic monomers include styrene, o-methylstyrene, n-methylstyrene, p-methylstyrene, α-methylstyrene, o-ethylstyrene, m-ethylstyrene, p-ethylstyrene, 2,4-dimethylstyrene, p-n-butylstyrene, p-t-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene and 3,4-dichlorostyrene.

Examples of (meth)acrylic ester monomers include hydrocarbon group-containing (meth)acrylic monomers such as methyl (meth)acrylate, ethyl (meth)acrylate, propyl (meth)acrylate, n-butyl (meth)acrylate, isobutyl (meth)acrylate, pentyl (meth)acrylate, hexyl (meth)acrylate, 2-ethylhexyl (meth)acrylate, octyl (meth)acrylate, nonyl (meth)acrylate, decyl (meth)acrylate, dodecyl (meth)acrylate, lauryl (meth)acrylate, stearyl (meth)acrylate, cyclohexyl (meth)acrylate, isobornyl (meth)acrylate, phenyl (meth)acrylate, toluyl (meth)acrylate and benzyl (meth)acrylate; fluorine-containing (meth)acrylic monomers such as 2,2,2-trifluoroethyl (meth)acrylate, 3,3,3-trifluoropropyl (meth)acrylate, 2-(perfluoroethyl)ethyl (meth)acrylate, 2-perfluoroethyl-2-perfluorobutylethyl (meth)acrylate, 2-perfluoroethyl (meth)acrylate, tetrafluoropropyl (meth)acrylate, perfluoromethyl (meth)acrylate, 1,1,1,3,3,3-hexafluoropropan-2-yl (meth)acrylate, 2-perfluoromethyl-2-perfluoroethylmethyl (meth)acrylate, 2-(perfluorohexyl)ethyl (meth)acrylate, 2-(perfluorodecyl)ethyl (meth)acrylate and 2-(perfluorohexadecyl)ethyl (meth)acrylate; alkylamino group-containing (meth)acrylic monomers such as N-propylaminoethyl acrylate, N-ethylaminopropyl (meth)acrylate, N-phenylaminoethyl (meth)acrylate and N-cyclohexylaminoethyl (meth)acrylate; silicon-containing (meth)acrylic monomers such as γ-(methacryloyloxypropyl)trimethoxysilane and γ-(methacryloyloxypropyl)dimethoxymethylsilane; alkoxy group-containing (meth)acrylic monomers such as (poly)ethylene glycol mono(meth)acrylate, 2-methoxyethyl (meth)acrylate and 3-methoxybutyl (meth)acrylate; (poly)alkylene glycol (meth)acrylic monomers such as (poly)propylene glycol mono(meth)acrylate; alkoxy(poly)alkylene glycol (meth)acrylic monomers such as methoxy(poly)ethylene glycol mono(meth)acrylate and methoxy(poly)propylene glycol mono(meth)acrylate; and 2-chloroethyl (meth)acrylate and methyl α-chloro(meth)acrylate.

Examples of vinyl carboxylate monomers include vinyl acetate, vinyl propionate, vinyl benzoate, vinyl butyrate, vinyl formate, vinyl valerate and vinyl pivalate.

Examples of N-vinyl compound monomers include N-vinylpyrrole, N-vinylcarbazole, N-vinylindole and N-vinylpyrrolidone.

Examples of olefinic monomers include ethylene and propylene. Examples of fluorinated olefinic monomers include vinyl fluoride, vinylidene fluoride, tetrafluoroethylene and hexafluoropropylene. Examples of conjugated diene monomers include butadiene and isoprene.

Examples of ionic functional group-containing monomers include those containing an anionic functional group such as a sulfonic acid group, phosphoric acid group or phenolic hydroxyl group (e.g., sodium p-styrenesulfonate) or a cationic functional group such as an amino, imidazole, pyridine or amidino group.

Unsaturated monomers having, for example, an alkylene oxide, carbonyl, ether, cyano, isocyanate, carbodiimide or oxazoline group may also be used as Unsaturated Monomer B.

Examples of carbonyl group-containing monomers include vinyl group-containing ketones such as vinyl methyl ketone, vinyl hexyl ketone and methyl isopropenyl ketone.

Examples of ether group-containing monomers include vinyl group-containing ethers such as vinyl methyl ether, vinyl ethyl ether and vinyl isobutyl ether.

Examples of cyano group-containing monomers include acrylonitrile, methacrylonitrile, hexenenitrile, 4-pentenenitrile and p-cyanostyrene.

Unsaturated Monomer B may be of one type used alone or of two or more types used in combination.

Of these, styrenic monomers, (meth)acrylic ester monomers and vinyl carboxylate monomers are especially preferred as Unsaturated Monomer B.

Recurring units derived from Unsaturated Monomer B are included in an amount, based on all recurring units, of from 0 to 95 mol%, preferably 20 to 94 mol%, more preferably from 50 to 93 mol%, and even more preferably from 60 to 92 mol%.

The crosslinked polymer particle of the invention, given the excellent crosslinking effects, is preferably synthesized using an epoxy group-containing monomer, and optionally a carboxyl group-containing monomer or amide group-containing monomer, as Unsaturated Monomer A, and using at least one type of monomer selected from among styrenic monomers, (meth)acrylic ester monomers and vinyl carboxylate monomers as Unsaturated Monomer B.

The crosslinked polymer particle of the invention preferably has characteristics that make the specific surface area relatively large, such as having fine irregularities at the particle surface or being porous. It is especially preferable for the particle to be porous.

The crosslinked polymer particle of the invention preferably has a specific surface area that satisfies the following formula.

$$SB/SD \geq 1.2$$

Here, SB is the actual specific surface area of each particle, and SD is the theoretical specific surface area of a spherical particle calculated from the volume mean particle size of each particle.

The ratio SB/SD is preferably at least 1.5, more preferably at least 1.8, and most preferably at least 2.0. Owing to this large specific surface area, the crosslinking reactions proceed efficiently at the time of reaction.

The actual specific surface area SB of the crosslinked polymer particle of the invention, although not particularly limited, is preferably from 0.1 to 30 $m^2$/g, more preferably from 0.5 to 20 $m^2$/g, and even more preferably from 1 to 10 $m^2$/g. The specific surface area SB is a value measured by the nitrogen gas adsorption method.

The crosslinked polymer particle of the invention is preferably a particle which has a water adsorption or oil adsorption of at least 50 mL per 100 g of particles, and has an affinity for aqueous systems, oil systems or both. The water adsorption or oil adsorption is more preferably at least 80 mL per 100 g of particles, even more preferably at least 100 g per 100 g of particles, and most preferably at least 120 mL per 100 g of particles.

The molecular weight of the polymer making up the particle is not particularly limited, although the weight-average molecular weight is typically from about 1,000 to about 3,000,000.

The crosslinked polymer particle of the invention has an excellent heat resistance. Specifically, the melting point is preferably at least 100°C, more preferably at least 120°C, even more preferably at least 150°C, and most preferably at least 200°C. Depending on the application or purpose for which the particle is to be used, it is even possible to produce a crosslinked polymer particle that melts at 250°C or more.

Another major characteristic of the crosslinked polymer particle of the invention is its excellent chemical resistance. Specifically, it does not dissolve easily at room temperature in water or organic solvents widely used in industrial applications, such as alcohols, hydrocarbons, acetic acid esters, ketones and ethers. In this invention, "chemical resistance" means that the crosslinked polymer particle of the invention, when added to at least one type of solvent selected from among ethanol, toluene, acetone, ethyl acetate, methyl ethyl ketone, dimethylformamide and dipropylene glycol and stirred for 30 minutes at 27°C, has a percent loss in weight of not more than 10 wt%.

The crosslinked polymer particle of the invention has both heat resistance and chemical resistance, but the resistance to hot chemicals is also a major feature. In this invention, "hot chemical resistance" means that the crosslinked polymer particle of the invention, when added to at least one type of solvent selected from among ethanol, toluene, ethyl acetate, methyl ethyl ketone, dimethylformamide and dipropylene glycol and stirred for 30 minutes at 70°C, has a percent loss in weight of not more than 10 wt%. Of these solvents, it is advantageous for the crosslinked polymer particle of the invention to have a hot chemical resistance with respect to preferably two or more, more preferably three or more, and most preferably all of the foregoing solvents. This enables applications to be developed in many fields.

[Method for Producing Elliptical, Needle-Shaped or Rod-Shaped Crosslinked Polymer Particle]

The crosslinked polymer particle of the invention can be produced by carrying out solution polymerization that entails heating a synthesis solution containing a mixed solvent of water, a hydrophilic organic solvent and a hydrophobic organic solvent, a high-molecular-weight stabilizer, a polymerization initiator and the above-described unsaturated monomer; and, at least following the start of heating, adjusting the pH of the synthesis solution to 5 or less or to 9 or more. After adjusting the synthesis solution pH to 5 or less or to 9 or more, it is preferable to maintain the pH at 5 or less or at 9 or more until reaction completion.

Specific examples of solution polymerization methods include suspension polymerization, emulsion polymerization, dispersion polymerization and seed polymerization methods, as well as combined methods based on these.

Suspension polymerization is a process in which a monomer and agents such as a polymerization initiator that are soluble in the monomer are mechanically agitated in a medium in which these do not readily dissolve, causing the polymerization reaction to proceed in a suspended state and causing polymer particles to separate out or form.

Emulsion polymerization is a process in which a medium such as water is mixed with a monomer and agents such as an emulsifying agent (surfactant) that are poorly soluble in the medium, along with which a polymerization initiator soluble in the medium is added, causing the polymerization reaction to proceed and polymer particles to separate out or form.

Dispersion polymerization is a process in which the polymerization reaction is made to proceed in a uniform solution of monomer, initiator, dispersion stabilizer and the like dissolved in a liquid medium within which the monomer dissolves but becomes insoluble with polymerization, causing polymer particles to separate out or form.

Seed polymerization is a polymerization process in which other particles serving as seeds are added beforehand at the time of polymerization and the polymerization reactions are carried out at the surface of these particles.

The crosslinked polymer particle of the invention can be obtained by these various types of solution polymerization, although a process based on suspension polymerization, emulsion polymerization, dispersion polymerization or a combination thereof is more preferred. With these methods, the seed particle preparation step required in seed polymerization can be omitted.

pH adjustment is carried out at least following the start of heating, although it may be carried out before the start of heating. The synthesis solution has a pH of preferably from 0 to 5 or from 9 to 14, more preferably from 0 to 4 or from 10 to 14, even more preferably from 0 to 3 or from 11 to 14, and most preferably from 0 to 2 or from 12 to 14. By thus shifting to the acidic or alkaline side, crosslinked particles can be obtained while allowing radical polymerization and crosslinking reactions by the reactive groups to proceed at the same time. When the reaction is made to proceed with the pH shifted to the acidic side, the polymerization reaction proceeds more stably and efficiently; hence, it is preferable for the pH to be from 0 to 5.

pH adjustment may be carried out by, for example, the gradual dropwise addition of a pH adjustor to the synthesis solution following the start of heating so as to shift the pH to the acidic or alkaline side. Alternatively, when the subsequently described persulfate is used as the polymerization initiator, because it breaks down during the polymerization reaction and forms an acid, the pH gradually decreases. In this case, a pH adjustor need not be added.

Examples of such pH adjustors include acids such as citric acid, tartaric acid, lactic acid, glycolic acid, hydrochloric acid, nitric acid, sodium citrate, sodium lactate, succinic acid, acetic acid, sodium acetate, fumaric acid, sulfuric acid, malic acid and phosphoric acid; and alkalis such as sodium hydroxide, potassium hydroxide, calcium hydroxide, magnesium hydroxide, sodium carbonate, potassium carbonate, calcium carbonate, ammonium carbonate, ammonia, morpholine, triethanolamine, diethanolamine, dimethylamine, diethylamine, trimethylamine and triethylamine.

By adjusting the pH of the synthesis solution to 5 or less or to 9 or more and carrying out solution polymerization, the crosslinking reactions proceed while the target elliptically shaped particle skeleton is being formed, and so monodispersed crosslinked polymer particles can be stably obtained with little agglomerates and impurities.

In this invention, the synthesis solution pH can be obtained by using a pH meter or pH test paper to measure the pH of the synthesis solution in an agitated state.

A mixed solvent of water, a hydrophilic organic solvent and a hydrophobic organic solvent is used as the solvent for synthesis. These solvents should be suitable ones selected from among ordinary solvents according to, for example, the starting materials to be used. In this invention, "hydrophilic organic solvent" refers to a solvent which maintains a uniform appearance as a mixed solvent with water. Also, "hydrophobic organic solvent" refers to a solvent for which, when gently mixed with an equal volume of pure water at one atmosphere ($1.013 \times 10^5$ Pa) and a temperature of 20°C, the resulting mixed liquid cannot maintain a uniform appearance after flow has subsided.

Examples of solvents that may be used include water, deionized water and distilled water; and hydrophilic organic solvents such as methanol, ethanol, 1-propanol, 2-propanol, ethylene glycol, propylene glycol, butylene glycol, dipropylene glycol, methyl cellosolve, ethyl cellosolve, propyl cellosolve, methyl cellosolve acetate, ethyl cellosolve acetate, methyl carbitol, ethyl carbitol, butyl carbitol, ethyl carbitol acetate, acetone, tetrahydrofuran, dimethyl formamide, N-methyl-2-pyrrolidone and acetonitrile. These may be used singly or two or more may be used in admixture.

Examples of hydrophobic organic solvents include higher alcohols such as 1-butanol, 2-butanol, isobutanol, t-butanol, 1-pentanol, 2-pentanol, 3-pentanol, 2-methyl-1-butanol, isopentyl alcohol, t-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as butyl cellosolve; polyethers such as polypropylene glycol and polybutylene glycol; ketones such as methyl ethyl ketone, methyl isobutyl ketone and cyclohexanone; esters such as ethyl acetate, butyl acetate, ethyl propionate and butyl carbitol acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methyl cyclopentane, methyl cyclohexane, ethyl cyclohexane, p-menthane, dicyclohexyl, benzene, toluene, xylene, ethylbenzene, liquid paraffin, mineral oil and heat transfer medium oils; siloxane compounds such as polydimethylsiloxane, polymethylphenylsiloxane, polydiphenylsiloxane and silicone oils; and halogenated hydrocarbons such as carbon tetrachloride, trichloroethylene, chlorobenzene and tetrabromoethane. These hydrophobic organic solvents may include modified compounds and copolymers and other modified polymer compounds that are substituted with carbon, nitrogen, oxygen, hydrogen, halogen or the like, within a range that does detract from the advantageous effects of the invention. Such hydrophobic organic solvents may be used singly or two or more may be used in combination.

Of the above hydrophobic organic solvents, the use of a hydrophobic organic solvent which does not react with the starting unsaturated monomer under the polymerization conditions and which has 8 or more carbon atoms is preferred. By having such a hydrophobic organic solvent be present within the reaction system, the dispersibility of the crosslinked polymer particles as they are formed can be enhanced, making more uniform control of the particle size possible.

This organic compound having 8 or more carbon atoms is not particularly limited, so long as it is a liquid--at least at the temperature at which the polymerization is made to proceed, and does not have an adverse influence on crosslinked polymer particle formation. However it is desirable to use an organic compound having a melting point of not above 80°C, preferably not above 60°C, more preferably not above 40°C, and still more preferably not above 30°C. Examples of such organic compounds include aliphatic or aromatic hydrocarbons such as n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene, ethylbenzene, liquid paraffin, mineral oil and heat transfer medium oils; siloxane compounds such as polydimethylsiloxane, polymethylphenylsiloxane, polydiphenylsiloxane and silicone oils; and polyethers such as polypropylene glycol and polybutylene glycol. The number of carbon atoms should be 8 or more, although taking into consideration the dispersion stability of the particles to be obtained, the number of carbon atoms is preferably at least 10, more preferably at least 12, and most preferably at least 15.

In addition, the molecular weight of the hydrophobic organic solvent is preferably at least 200, more preferably at least 300, even more preferably at least 500, and most preferably at least 1,000. By thus using a hydrophobic organic solvent having a high molecular weight, the solvent also carries out a dispersant-like role, minimizing sticking and agglomeration of the particles and making it possible to obtain crosslinked polymer particles that are stably monodispersed and have a controlled particle size.

In this invention, "molecular weight" refers to, in the case of high-molecular-weight compounds, the weight-average molecular weight. The weight-average molecular weight is a polystyrene-equivalent measured value obtained by gel permeation chromatography.

High-molecular-weight compounds having recurring units are preferred as the hydrophobic organic solvent with a molecular weight of 200 or more. Specific examples include siloxane compounds such as polydimethylsiloxane, polymethylphenylsiloxane, polydiphenylsiloxane and silicone oils; polyethers such as polypropylene glycol and polybutylene glycol; and aliphatic or aromatic hydrocarbons such as liquid paraffin and heat transfer medium oils. These high-molecular-weight compounds are more preferably high-molecular-weight compounds which are water-soluble in a low-molecular-weight state and exhibit hydrophobicity as the molecular weight increases, or hydrophobic organic solvents obtained by polymerizing a monomer having a polar group at the interior of the molecule. By having such polar groups at the interior of the molecule, the subsequently described high-molecular-weight stabilizer readily disperses uniformly within the solvent, further contributing to particle stability. Examples of such polar groups include hydroxyl, ether and carbonyl groups.

Examples of such preferred hydrophobic organic solvents include polyethers such as polypropylene glycol and polybutylene glycol; and siloxane compounds such as polydimethylsiloxane, polymethylphenylsiloxane, polydiphenylsiloxane and silicone oils.

The mixing ratio of the water, the hydrophilic organic solvent and the hydrophobic organic solvent, expressed as a weight ratio, is preferably from 99:0.5:0.5 to 25:55:20, more preferably from 98:1:1 to 35:50:15, and even more preferably from 97:2:1 to 45:45:10.

By using such a mixed solvent, there arises a "fuzzy" state within which co-exist an emulsified layer (bottom layer, or water-rich layer), a dissolved layer (intermediate layer, or hydrophilic solvent-rich layer) and a separated layer (top layer, or hydrophobic solvent-rich layer) that appear when the solvent is at rest, and the polymerization reaction also is thought to proceed with the unsaturated monomer dissolved in each of these layers. It is thought that, in cases where the polymerization reaction is carried out in a solvent system that forms this fuzzy state, when polymerization is induced by an initiator, the solvent dissolution balance due to heat breaks down and the tension at the particle separation boundary changes, enabling elliptical, needle-shaped or rod-shaped polymer particles to be obtained in a stably dispersed state; along with this, setting the pH of the synthesis solution to a specific value promotes the crosslinking reaction, enabling crosslinked polymer particles to be obtained.

The high-molecular-weight stabilizer is exemplified by various types of hydrophobic or hydrophilic stabilizers, including polyethylene glycol and polystyrene derivatives such as polyhydroxystyrene, polystyrene sulfonic acid, hydroxystyrene-(meth)acrylic ester copolymers, styrene-(meth)acrylic ester copolymers and styrene-hydroxystyrene-(meth)acrylic ester copolymers; poly(meth)acrylic acid and poly(meth)acrylic acid derivatives such as poly(meth)acrylamide, polyacrylonitrile, polyethyl (meth)acrylate and polybutyl (meth)acrylate; polyethers and derivatives thereof, such as poly(methyl vinyl ether), poly(ethyl vinyl ether), poly(butyl vinyl ether), poly(isobutyl vinyl ether) and poly(hexyl vinyl ether); cellulose and cellulose derivatives such as methyl cellulose, cellulose acetate, cellulose nitrate, hydroxymethyl cellulose, hydroxyethyl cellulose, hydroxypropyl cellulose and carboxymethyl cellulose; polyvinyl alcohol, polyvinyl butyral, polyvinyl formal and polyvinyl acetate derivatives such as polyvinyl acetate; nitrogen-containing polymer derivatives such as polyvinyl pyridine, polyvinyl pyrrolidone, polyethyleneimine and poly-2-methyl-2-oxazoline; and polyvinyl halide derivatives such as polyvinyl chloride and polyvinylidene chloride. These may be of one type used alone or two or more may be used in combination.

The high-molecular-weight stabilizer is preferably included in a suitable amount of from 0.01 to 50 wt%, based on the starting unsaturated monomer.

Various known polymerization initiators may be used as the polymerization initiator. Examples include the following water-soluble or ionic polymerization initiators:

persulfates such as ammonium persulfate, sodium persulfate and potassium persulfate; and azo-type initiators such as 2,2'-azobis[2-methyl-N-(2-hydroxyethyl)propionamide],
2,2'-azobis(2-amidinopropane) dihydrochloride,
2,2'-azobis(2-methyl-N-phenylpropionamidine) dihydrochloride,
2,2'-azobis[N-(4-chlorophenyl)-2-methylpropionamidine] dihydrochloride,
2,2'-azobis[N-(4-hydroxyphenyl)-2-methylpropionamidine] dihydrochloride,
2,2'-azobis[N-(4-aminophenyl)-2-methylpropionamidine] tetrahydrochloride,
2,2'-azobis[2-methyl-N-(phenylmethyl)propionamidine] dihydrochloride,
2,2'-azobis[2-methyl-N-2-propenylpropionamidine] dihydrochloride,
2,2'-azobis[N-(2-hydroxyethyl)-2-methylpropionamidine] dihydrochloride,
2,2'-azobis[2-(5-methyl-2-imidazolin-2-yl)propane] dihydrochloride,
2,2'-azobis[2-(2-imidazolin-2-yl)propane] dihydrochloride,
2,2'-azobis[2-(4,5,6,7-tetrahydro-1H-1,3-diazepin-2-yl)propane] dihydrochloride,
2,2'-azobis[2-(3,4,5,6-tetrahydropyrimidin-2-yl)propane] dihydrochloride,
2,2'-azobis[2-(5-hydroxy-3,4,5,6-tetrahydropyrimidin-2-yl)propane] dihydrochloride,
2,2'-azobis{2-[1-(2-hydroxyethyl)-2-imidazolin-2-yl] propane} dihydrochloride,
2,2'-azobis-2-cyanopropane-1-sulfonic acid disodium salt, and
sodium 4,4'-azobis(4-cyanopentanoate).

Examples of oil-soluble polymerization initiators include peroxides such as benzoyl peroxide, cumene hydroperoxide and t-butyl hydroperoxide; and azo compounds such as azobisisobutyronitrile, azobismethylbutyronitrile, azobisisovaleronitrile, 2,2'-azobis(dimethyl isobutyrate), 2,2'-azobis(N-butyl-2-methylpropionamide), 4,4'-azobis(4-cyanopentanoic acid), 2,2'-azobis(2-amidinopropane) dihydrochloride and 2,2'-azobis(N,N'-dimethylene isobutylamidine) dihydrochloride.

These polymerization initiators may be used singly or two or more may be used in admixture. It is preferable for the content of the radical polymerization initiator to be generally from 0.01 to 50 parts by weight per 100 parts by weight of the starting unsaturated monomer.

Of these, by using an initiator such as a persulfate, not only does this compound function as a radical initiator, an acid forms by hydrolysis or the like, making it possible to adjust the pH of the synthesis solution without having to separately add a pH adjustor. The preferred amount of addition is from 5 to 30 wt%.

It is possible to impart characteristics such as fine surface irregularities, porosity and a large specific surface area to the crosslinked polymer particles by suitably adjusting the water, hydrophilic organic solvent and hydrophobic organic solvent ingredients and composition. The particle surface and interior can be suitably modified in this way.

In this invention, by carrying out the above types of adjustments in the solvent composition, it is possible to more stably control the particle size and aspect ratio, the size of fine surface irregularities and the porosity of the crosslinked polymer particles, enabling a good balance in performance attributes such as the water absorption and oil absorption to be achieved in accordance with the intended use.

When producing the crosslinked polymer particles of the invention, an emulsifying agent (surfactant) may be optionally included in a suitable amount of from 0.01 to 50 wt%, based on the starting unsaturated monomer.

The emulsifying agent (surfactant) is exemplified by anionic emulsifying agents, including alkyl sulfates such as sodium dodecyl sulfate, alkylbenzenesulfonates such as sodium dodecylbenzenesulfonate, alkylnaphthalenesulfonates, fatty acid salts, alkyl phosphates and alkyl sulfosuccinates; cationic emulsifying agents such as alkylamine salts, quaternary ammonium salts, alkyl betaines and amine oxides; and nonionic emulsifying agents such as polyoxyethylene alkyl ethers, polyoxyethylene alkyl allyl ethers, polyoxyethylene alkyl phenyl ethers, sorbitan fatty acid esters, glycerol fatty acid esters, sucrose fatty acid esters and polyoxyethylene fatty acid esters. These may be used singly or two or more may be used in combination.

By adding an emulsifying agent, the length and breadth of the crosslinked polymer particles can be controlled. In addition, it is preferable to include at least one emulsifying agent that is solid at room temperature.

A suitable polyfunctional unsaturated monomer may be included as a crosslinking coagent at the time of the polymerization reaction, within a range that does not detract from the characteristics of the crosslinked polymer particle of the invention. Depending on the heat-resistant or chemical-resistant application, synergistic effects are sometimes obtained even with a small amount. Examples of crosslinking agents include aromatic divinyl compounds such as divinylbenzene, divinylbiphenyl and divinylnaphthalene; (poly)alkylene glycol di(meth)acrylates such as (poly)ethylene glycol di(meth)acrylate, (poly)propylene glycol di(meth)acrylate and (poly)tetramethylene glycol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate and 2-methyl-1,8-octanediol di(meth)acrylate; alkanediol di(meth)acrylates such as 1,6-hexanediol di(meth)acrylate, 1,8-octanediol di(meth)acrylate, 1,9-nonanediol di(meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate and 2-methyl-1,8-octanediol di(meth)acrylate; polyfunctional (meth)acrylates such as glycerol di(meth)acrylate, pentaerythritol tri(meth)acrylate, pentaerythritol tetra(meth)acrylate, pentaerythritol di(meth)acrylate, pentaerythritol tetra(meth)acrylate, glycerol acryloxy di(meth)acrylate, ethoxylated cyclohexane dimethanol di(meth)acrylate, ethoxylated bisphenol A di(meth)acrylate, tricyclodecane dimethanol di(meth)acrylate, propoxylated ethoxylated bisphenol A di(meth)acrylate, 1,1,1-trishydroxymethylethane di(meth)acrylate, 1,1,1-trishydroxymethylethane tri(meth)acrylate, 1,1,1-trishydroxymethylpropane tri(meth)acrylate, caprolactone-modified dipentaerythritol hexa(meth)acrylate, caprolactone-modified hydroxypivalate neopentyl glycol di(meth)acrylate, polyester (meth)acrylate and urethane (meth)acrylate; and compounds such as N,N-divinylaniline, divinyl ether, divinylsulfide and divinylsulfone. These may be used singly or two or more may be used in combination.

The content of starting unsaturated monomer in the synthesis solution is preferably set to from 1 to 80 wt%, more preferably from 5 to 50 wt%, and even more preferably from 10 to 30 wt%, of the overall synthesis solution. At a starting unsaturated monomer content greater than 80 wt%, agglomerates increase and obtaining, in a monodispersed state, a high yield of polymer particles having the above properties may be difficult. On the other hand, at a content below 1 wt%, completion of the reaction takes a long time and is impractical from an industrial standpoint.

The reaction temperature during polymerization varies depending on the type of solvent used, and therefore cannot be strictly specified, but is typically from about 10°C to about 200°C, preferably from 30 to 130°C, and more preferably from 40 to 90°C.

The reaction time is not particularly limited, so long as it is the time needed for the intended reaction to go substantially to completion, and is governed largely by such factors as the type and content of the unsaturated monomer, the viscosity and concentration of the solution, and the intended particle size. For example, at from 40 to 90°C, the reaction time is typically from 1 to 72 hours, and preferably from about 2 hours to about 24 hours.

Depending on such considerations as the intended use of the resulting particles, a catalyst (reaction accelerator) may be included at the time of the polymerization reaction. The content may be set to a suitable amount that does not adversely affect the particle properties, such as from 0.01 to 20 wt% of the total weight of the polymerization ingredients.

The catalyst is not particularly limited so long as it is a positive catalyst, and may be suitably selected from among known catalysts and used. Examples include tertiary amines such as benzyldimethylamine, triethylamine, tributylamine, pyridine and triphenylamine; quaternary ammonium compounds such as triethylbenzylammonium chloride and tetramethylammonium chloride; phosphines such as triphenylphosphine and tricyclophosphine; phosphonium compounds such as benzyl trimethylphosphonium chloride; imidazole compounds such as 2-methylimidazole and 2-methyl-4-ethylimidazole; alkali metal hydroxides such as potassium hydroxide, sodium hydroxide and lithium hydroxide; alkali metal carbonates such as sodium carbonate and lithium carbonate; alkali metal salts of organic acids; and halides that exhibit Lewis acid properties, such as boron trichloride, boron trifluoride, tin tetrachloride and titanium tetrachloride, or complex salts thereof. These may be used singly or two or more may be used in combination.

At the time of the polymerization reaction, in order to adjust the size, shape, quality and the like of the resulting crosslinked polymer particles, it is also possible to add a compound that is soluble in water or another polar solvent and undergoes electrolytic dissociation into cations and anions, such that the solution exhibits electrical conductivity.

Such compounds are exemplified by salts, inorganic acids, inorganic bases, organic acids, organic bases and ionic liquids. The content is a suitable amount that does not adversely affect the particle properties, and may be set to, for example, from 0.01 to 80 wt% of the total weight of the polymerization ingredients.

By varying the types and weight ratios of the unsaturated monomer, high-molecular-weight stabilizer and solvent, and the weight ratios of the optionally used dispersant and emulsifying agent, crosslinked polymer particles of differing particle sizes, aspect ratios, shapes and the like can be more stably produced in a monodispersed state.

[Uses of Elliptical, Needle-Shaped or Rod-Shaped Crosslinked Polymer Particle]

The crosslinked polymer particle of the invention has excellent heat and chemical resistances, and thus can be used in polymer molded or formed articles such as plastics, containers, paints, paint films, fibers and building materials. Also, because it is effective as well in terms of UV scattering properties, it can be used for the protection of UV-sensitive contents, such as in filters, packaging materials, containers, paints, paint films, inks, fibers, building materials, recording media, image displaying devices and solar cell covers, and can also check the decomposition of compounds unstable to light.

In addition, compared with conventional spherical particles, the crosslinked polymer particle of the invention can increase the strength of molded or formed articles. Hence, because the strength of molded or formed articles can be maintained even with high loadings of particles, use in light-diffusing plates, light-diffusing sheets and thermally cavitated products having pores that utilize optical characteristics is also possible.

The crosslinked polymer particle of the invention may be dispersed in water, a hydrophilic organic solvent, a hydrophobic organic solvent or a mixed solvent thereof and used as a dispersion. The hydrophilic organic solvent and hydrophobic organic solvent are exemplified by the same solvents mentioned above in connection with the polymer particle production method.

The crosslinked polymer particle of the invention may be used as an additive in liquids and formed articles such as paint films, film, sheet stock and paper. The crosslinked polymer particle-containing composition of the invention may be widely used in, for example, light scattering agents and optical filter materials, colorants, cosmetics, absorbents, adsorbents, inks, adhesives, electromagnetic shielding materials, fluorescence sensors, biological markers, recording media, recording elements, polarizing materials, drug supports for drug delivery systems (DDS), biosensors, DNA chips, diagnostic agents and thermally cavitated products having pores.

Also, by incorporating the crosslinked polymer particle of the invention into a precursor, and carrying out firing treatment such as curing, carbonization or sintering, a thermally cavitated product having particle-shaped pores can be produced.

Using window glass products or interior decoration products such as curtains and wall materials to block light or ultraviolet radiation from entering into a room, car or the like is useful not only for preventing sunburn and other adverse effects to the human body, but also for preventing the deterioration of decorative objects within the room or car.

The crosslinked polymer particle of the invention is suitable as an additive for cosmetics. Expansion into thermoforming applications and applications that use a large amount of organic solvent where addition has hitherto been difficult is now possible while retaining such desirable features of the crosslinked polymer particle of the invention as its low weight and its light scattering properties, tactile qualities, flowability and solution dispersibility. The crosslinked polymer particle of the invention, owing to its distinctive shape, has an adhesive strength differing from that of ordinary spherical particles, and is thus effective for improving both the bonding strength of pressed compacts of cosmetic foundation and also the holding power following application. In addition, the optical characteristics make the skin appear lighter and can enhance the covering power due to a shading effect. Also, due to the slip properties particular to the particle shape, spreadability over the skin is excellent and furrows in the skin texture are finely filled, making wrinkles and pores inconspicuous, and the flowability of the overall product can be freely controlled. Also, the adhesive strength and holding power can be utilized to increase the amount of polymer addition in the overall product, enabling the discovery of entirely new cosmetic effects. The amount of addition, based on the product contents, is preferably from 0.1 to 50 wt%, and more preferably from 0.5 to 30 wt%. This amount may be suitably adjusted according to the intended use and purpose, such as enhancing the light scattering properties (e.g., the UV scattering effect and the shading effect), flowability, moldability and adhesion, and the finished look. According to studies by the inventors, as an additive for cosmetics, the addition of 1 to 20 wt% is especially preferred. Suitable adjustment and use in combination with commercially available particles is also possible.

Cosmetics in which the advantageous effects are high include in particular skin care products, hair products, antiperspirants, makeup products, UV protection products and scented products. Specific examples include base cosmetics such as milky emulsions, creams, lotions, calamine lotion, sunscreens, makeup base, suntan lotions, aftershave lotions, preshave lotions, packs, cleansing materials, facial cleansers, cosmetics for acne, and essences; makeup cosmetics such as foundation, face powder, mascara, eye shadow, eyeliner, eyebrow, cheek, nail color, lip cream and lipstick; and also shampoos, rinses, conditioners, hair colors, hair tonics, setting agents, body powders, hair growth promoters, deodorants, depilatories, soaps, body shampoos, bath preparations, hand soaps and perfumes. The form of the product is not particularly limited and includes, for example, liquids, emulsions, creams, solids, pastes, gels, powders, multi-layer preparations, mousses and sprays. Useful effects can be expected of the crosslinked polymer particle as an additive in these cosmetics.

The crosslinked polymer particle of the invention can be utilized as additives for printing inks that may be used in, for example, screen printing, offset printing, process printing, gravure printing, pad printing, coaters and inkjet printing; as additives for writing implement inks in marking pens, ballpoint pens, fountain pens, calligraphy pens and magic markers; and as additives for writing materials such as crayons, artist's paints and erasers.

The crosslinked polymer particle of the invention is suitable as an additive for paints that may be used in brush painting, spray painting, electrostatic spray painting, electrodeposition painting, flow coating, roller coating and dip coating. For example, it is suitable as an additive for paints and coatings that may be used on transportation equipment such as automobiles, railway cars, helicopters, ships, bicycles, snowmobiles, ropeways, lifts, hovercrafts and motorcycles; building members such as window sashes, shutters, cisterns, doors, balconies, outside panels for construction, roofing, staircases, skylights and concrete walls; the exterior walls and interior finish on the inside and outside of buildings; roadway members such as guardrails, pedestrian bridges, sound insulating walls, road signs, highway sidewalls, elevated railway bridges, and bridges; industrial plant members such as tanks, pipes, towers and smokestacks; agricultural facilities such as PVC and other types of greenhouses, silos and agricultural sheeting; telecommunications facilities such as utility poles, transmission towers and parabolic antennas; electrical equipment such as electrical service boxes, lighting equipment, outdoor air conditioners, washing machines, refrigerators and electric ranges, as well as covers for these; and other articles such as monuments, gravestones, paving materials, windscreens, waterproof sheeting and curing sheets for construction.

The form of the paint is exemplified by not only solvent-based paints, but also water-dispersed paints, non-water-dispersed paints, powder paints and electrodeposition paints, and may be suitably selected as needed.

<u>EXAMPLES</u>

Synthesis Examples, Working Examples and Comparative Examples are given below by way of illustration, although the invention is not limited to these Examples. Evaluations in the Working Examples and Comparative Examples were carried out by the following methods.

(1) Aspect Ratio of Polymer Particles

A scanning electron microscope (S-4800, from Hitachi High Technologies Corporation; referred to below as "SEM") was used to capture photographs at a magnification at which particle measurement is possible (300 to 30,000×), thereby rendering the elliptical, needle-shaped or rod-shaped crosslinked polymer particles obtained into two-dimensional images (elliptical, needle-shaped or rod-shaped crosslinked polymer particles typically maintain a state in which the long axis direction is horizontally oriented). In the images, 100 particles were randomly sampled and the length (L) and breadth (D) of each particle were measured, the aspect ratio (L/D) for the particle was calculated, and the average aspect ratio ($P_{AV}$) was determined.

The average length ($L_{AV}$) and average breadth ($D_{AV}$) of the particles were similarly calculated after measuring the length (L) and breadth (D) of 100 randomly sampled particles.

(2) Volume Mean Particle Size (MV) of Polymer Particles

The volume mean particle size was measured using the MICROTRACK MT3000 (Nikkiso Co., Ltd.).

(3) Specific Surface Area (SS)

The specific surface area was measured by the nitrogen gas adsorption method using an automatic specific area/pore size distribution measuring instrument (BELSORP-max, from Bel Japan, Inc.).

(4) Theoretical Specific Surface Area (SD)

Letting 2r (m) be the volume mean particle size, r (m) be the radius and G (g/m$^3$) be the density of an elliptical, needle-shaped or rod-shaped crosslinked polymer particle, the surface area S' (m$^2$) and volume V (m$^3$) of a spherical particle of radius r (m) is expressed as follows.

$$\text{Surface area of spherical particle of radius r (m): } S' \text{ (m}^2) = 4\pi r^2$$

$$\text{Volume of spherical particle of radius r (m): } V\ (m^3) = 4\pi r^3/3$$

In this case, the number N of particles contained in one gram of particles is expressed as follows.

$$\text{Number of particles contained in one gram of particles: } N = 1/VG$$

Therefore, the theoretical specific surface area SD ($m^2/g$) of spherical particles calculated from elliptical, needle-shaped or rod-shaped polymer particles is expressed as follows.

$$SD\ (m^2/g) = S'N = S'/VG = 3/rG$$

(5) Measurement of Water Absorption

A dried polymer particle powder was dispersed in water to a concentration of about 2 wt% and left at rest for one day, following which it was re-dispersed and then filtered under reduced pressure using a glass filter. The glass filter used in filtration was then subjected to 30 minutes of centrifugation at 3,000 rpm using a centrifuge (CR-20GII, from Hitachi High Technologies Corporation), following which the resulting polymer particle powder was dried. The weight of the powder before and after drying was measured, and the difference in weight was treated as the amount of absorbed water.

(6) Measurement of Oil Absorption

Oil absorption was measured in accordance with the boiled linseed oil method described in JIS K 5101.

(7) pH Measurement

The pH was determined by visually judging the change in color using pH test paper (from Whatman).

[1] Synthesis of Crosslinked Polymer Particles

[Working Example 1]

A synthesis solution was prepared by charging the compounds shown below all at once into a 2,000 mL flask. The solution was then stirred at room temperature for one hour. The liquid phase was in a state where an aqueous phase portion, an emulsified phase portion and an oil phase portion are intermingled. The pH was measured and found to be 7. Next, under a stream of nitrogen, the solution was set to an oil bath temperature of 80°C and heating and stirring (400 rpm) were begun. After 8 hours of heating and stirring (400 rpm), a methyl methacrylate-glycidyl methacrylate copolymer particle dispersion was obtained. In the course of this step, when the internal temperature reached 40°C, 1 mol/L hydrochloric acid was added dropwise for 15 minutes as a pH adjustor to the synthesis solution until the pH became 1. The pH at the time of reaction completion was also 1.

| | |
|---|---|
| Water | 1,054.0 g |
| Methanol | 186.0 g |
| Polypropylene glycol (#1000) | 23.3 g |
| Polyvinyl pyrrolidone (K-15) | 28.0 g |
| Sorbitan monooleate | 7.2 g |
| Azobisisobutyronitrile | 28.6 g |
| Methyl methacrylate | 306.0 g |
| Glycidyl methacrylate | 54.0 g |

The resulting particle dispersion was transferred to a separate 3,000 mL flask and the synthesis vessel (the flask and the stirring element) was checked for deposits; a clean state was observed with substantially no agglomerate.
Next, using a known suction filtration apparatus, the separately transferred particle dispersion was repeatedly (5 times) washed with methanol and filtered, and then vacuum dried, giving Polymer Particle A1. The makeup (molar ratio) of the respective recurring units in Polymer Particle A1 was methyl methacrylate : glycidyl methacrylate = 89:11.

One hundred of the resulting particles were randomly sampled and their shapes examined with the SEM. The particles were found to be elliptical, needle-shaped or rod-shaped polymer particles. The $L_{AV}$ was 18.2 $\mu$m, and the $P_{AV}$ was 6.5.

The particles were checked for extraneous matter, but substantially no deformed matter such as agglomerates and mutually sticking particles was observed.

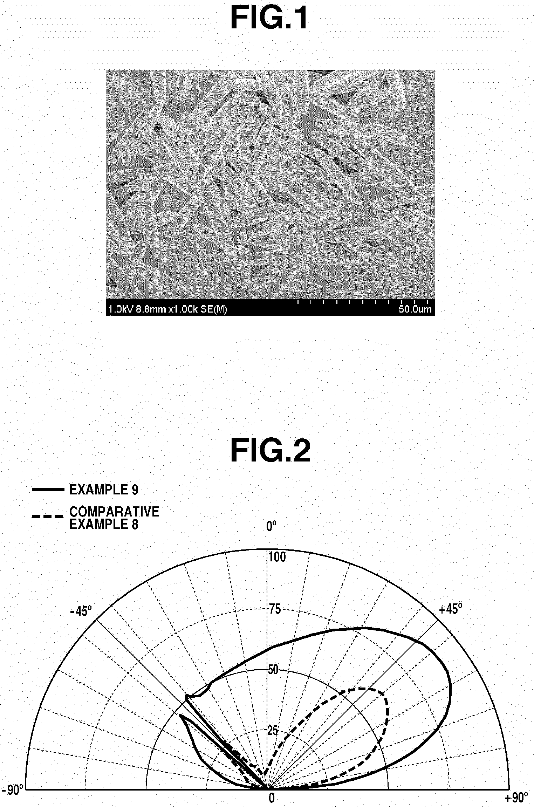

FIG. 1 shows a SEM micrograph of the resulting particles.

A portion of the resulting particles was measured with a Fourier transform infrared spectrophotometer (FT-IR8200PC, from Shimadzu Corporation). The peak at a wave number of 910 (cm$^{-1}$) attributable to epoxy groups was confirmed to be smaller than before synthesis.

[Working Example 2]

Aside from changing the pH adjustor to 1 mol/L sodium hydroxide and adjusting the pH at the time of synthesis to 12, a methyl methacrylate-glycidyl methacrylate copolymer particle dispersion was obtained in the same way as in Working Example 1. The pH at the time of reaction completion was also 12.

The resulting particle dispersion was transferred to a separate 3,000 mL flask and the synthesis vessel (the flask and the stirring element) was checked for deposits; a clean state was observed with substantially no agglomerate.

Next, using a known suction filtration apparatus, the separately transferred particle dispersion was repeatedly (5 times) washed with methanol and filtered, and then vacuum dried, giving Polymer Particle A2. The makeup (molar ratio) of the respective recurring units in Polymer Particle A2 was methyl methacrylate : glycidyl methacrylate = 89:11.

One hundred of the resulting particles were randomly sampled and their shapes examined with the SEM. The particles were found to be elliptical, needle-shaped or rod-shaped polymer particles. The $L_{AV}$ was 16.1 $\mu$m, and the $P_{AV}$ was 8.1.

The particles were checked for extraneous matter, but substantially no deformed matter such as agglomerates and mutually sticking particles was observed.

A portion of the resulting particles was measured with a Fourier transform infrared spectrophotometer. The peak attributable to epoxy groups at a wave number of 910 (cm$^1$) was confirmed to be smaller than before synthesis.

[Working Example 3]

A synthesis solution was prepared by charging the compounds shown below all at once into a 2,000 mL flask. The solution was then stirred at room temperature for one hour. The liquid phase was in a state where an aqueous phase portion, an emulsified phase portion and an oil phase portion are intermingled. Next, under a stream of nitrogen, the solution was set to an oil bath temperature of 80°C and heating and stirring (400 rpm) were begun. After 8 hours of heating and stirring (400 rpm), a styrene-methacrylic acid-glycidyl methacrylate copolymer particle dispersion was obtained. The pH of the synthesis solution before the start of heating was 7, the pH 2 hours after the start of heating was 2, and the pH at the time of reaction completion was 1.

| | |
|---|---|
| Water | 1,085.0 g |
| Methanol | 155.0 g |
| Polypropylene glycol (#2000) | 15.1 g |
| Polyvinyl pyrrolidone (K-15) | 25.0 g |
| Sucrose ester of lauric acid | 7.0 g |
| Ammonium persulfate | 28.8 g |
| Styrene | 144.0 g |
| Methacrylic acid | 144.0 g |
| Glycidyl methacrylate | 72.0 g |

The resulting particle dispersion was transferred to a separate 3,000 mL flask and the synthesis vessel (the flask and the stirring element) was checked for deposits; a clean state was observed with substantially no agglomerates.

Next, using a known suction filtration apparatus, the separately transferred particle dispersion was repeatedly (5 times) washed with methanol and filtered, and then vacuum dried, giving Polymer Particle A3. The makeup (molar ratio) of the respective recurring units in Polymer Particle A3 was styrene : methacrylic acid : glycidyl methacrylate = 37:44:19.

One hundred of the resulting particles were randomly sampled and their shapes examined with the SEM. The particles were found to be elliptical, needle-shaped or rod-shaped polymer particles. The $L_{AV}$ was 31.6 $\mu$m, and the $P_{AV}$ was 10.5.

The particles were checked for extraneous matter, but substantially no deformed matter such as agglomerates and mutually sticking particles was observed.

A portion of the resulting particles was measured with a Fourier transform infrared spectrophotometer, whereupon the peak at a wave number of 910 (cm$^{-1}$) attributable to epoxy groups was confirmed to be smaller than before synthesis.

[Working Example 4]

A synthesis solution was prepared by charging the compounds shown below all at once into a 2,000 mL flask. The solution was then stirred at room temperature for one hour. The liquid phase was in a state where an aqueous phase portion, an emulsified phase portion and an oil phase portion are intermingled. Next, under a stream of nitrogen, the solution was set to an oil bath temperature of 80°C and heating and stirring (400 rpm) were begun. After 8 hours of heating and stirring (400 rpm), a styrene-glycidyl methacrylate copolymer particle dispersion was obtained. The pH of the synthesis solution before the start of heating was 7, the pH 2 hours after the start of heating was 2, and the pH at the time of reaction completion was 1.

| | |
|---|---|
| Water | 896.0 g |
| Methanol | 384.0 g |
| Polypropylene glycol (#1000) | 25.5 g |
| Polyvinyl pyrrolidone (K-30) | 10.5 g |
| Polyethylene oxide (Mw, 100,000) | 8.0 g |
| Ammonium persulfate | 25.7 g |
| Styrene | 171.0 g |
| Glycidyl methacrylate | 114.0 g |

The resulting particle dispersion was transferred to a separate 3,000 mL flask and the synthesis vessel (the flask and the stirring element) was checked for deposits; a clean state was observed with substantially no agglomerates.

Next, using a known suction filtration apparatus, the separately transferred particle dispersion were repeatedly washed (5 times) with methanol and filtered, and then vacuum dried, giving Polymer Particle A4. The makeup (molar ratio) of the respective recurring units in Polymer Particle A4 was styrene : glycidyl methacrylate = 67:33.

One hundred of the resulting particles were randomly sampled and their shapes examined with the SEM. The particles were found to be elliptical, needle-shaped or rod-shaped polymer particles. The $L_{AV}$ was 12.4 $\mu$m, and the $P_{AV}$ was 8.7.

The particles were checked for extraneous matter, but substantially no deformed matter such as agglomerates and mutually sticking particles was observed.

A portion of the resulting particles was measured with a Fourier transform infrared spectrophotometer. The peak at a wave number of 910 (cm$^{-1}$) attributable to epoxy groups was confirmed to be smaller than before synthesis.

[Working Example 5]

A synthesis solution was prepared by charging the compounds shown below all at once into a 2,000 mL flask. The solution was then stirred at room temperature for one hour. The liquid phase was in a state where an aqueous phase portion, an emulsified phase portion and an oil phase portion are intermingled. The pH was measured and found to be 8. Next, under a stream of nitrogen, the solution was set to an oil bath temperature of 85°C and heating and stirring (400 rpm) were begun. After 15 hours of heating and stirring (400 rpm), a styrene-methacrylamide-glycidyl methacrylate copolymer particle dispersion was obtained. In the course of this step, when the internal temperature reached 40°C, triethylamine was added dropwise for 15 minutes as a pH adjustor to the synthesis solution until the pH became 12. The pH at the time of reaction completion was also 12.

| | |
|---|---|
| Water | 1,273.0 g |
| Ethanol | 67.0 g |
| Polypropylene glycol (#3000) | 12.0 g |
| Polyvinyl pyrrolidone (K-15) | 25.0 g |

(continued)

| | |
|---|---|
| Polyethylene glycol (PEG 20000) | 12.5 g |
| Sorbitan monooleate | 14.5 g |
| Azobisisobutyronitrile | 12.6 g |
| Styrene | 105.0 g |
| Methacrylamide | 63.0 g |
| Glycidyl methacrylate | 42.0 g |

The resulting particle dispersion was transferred to a separate 3,000 mL flask and the synthesis vessel (the flask and the stirring element) was checked for deposits; a clean state was observed with substantially no agglomerates.

Next, using a known suction filtration apparatus, the separately transferred particle dispersion was repeatedly (5 times) washed with methanol and filtered, and then vacuum dried, giving Polymer Particle A5. The makeup (molar ratio) of the respective recurring units in Polymer Particle A5 was styrene : methacrylamide : glycidyl methacrylate = 49:36:15.

One hundred of the resulting particles were randomly sampled and their shapes examined with the SEM. The particles were found to be elliptical, needle-shaped or rod-shaped polymer particles. The $L_{AV}$ was 24.8 $\mu$m, and the $P_{AV}$ was 7.2.

The particles were checked for extraneous matter, but substantially no deformed matter such as agglomerates and mutually sticking particles was observed.

A portion of the resulting particles was measured with a Fourier transform infrared spectrophotometer. The peak at a wave number of 910 (cm$^{-1}$) attributable to epoxy groups was confirmed to be smaller than before synthesis.

[Working Example 6]

A synthesis solution was prepared by charging the compounds shown below all at once into a 2,000 mL flask. The solution was then stirred at room temperature for one hour. The liquid phase was in a state where an aqueous phase portion, an emulsified phase portion and an oil phase portion are intermingled. Next, under a stream of nitrogen, the solution was set to an oil bath temperature of 80°C and heating and stirring (300 rpm) were begun. After 8 hours of heating and stirring (300 rpm), a methyl methacrylate-glycidyl methacrylate copolymer particle dispersion was obtained. The pH of the synthesis solution before the start of heating was 7, the pH 2 hours after the start of heating was 2, and the pH at the time of reaction completion was 1.

| | |
|---|---|
| Water | 1,254.0 g |
| Methanol | 66.0 g |
| Polypropylene glycol (#1000) | 18.0 g |
| Polyvinyl pyrrolidone (K-15) | 25.0 g |
| Sucrose ester of lauric acid | 6.5 g |
| Ammonium persulfate | 27.1 g |
| Methyl methacrylate | 255.0 g |
| Glycidyl methacrylate | 63.9 g |

The resulting particle dispersion was transferred to a separate 3,000 mL flask and the synthesis vessel (the flask and the stirring element) was checked for deposits; a clean state was observed with substantially no agglomerates.

Next, using a known suction filtration apparatus, the separately transferred particle dispersion was repeatedly (5 times) washed with methanol and filtered, and then vacuum dried, giving Polymer Particle A6. The makeup (molar ratio) of the respective recurring units in Polymer Particle A6 was methyl methacrylate : glycidyl methacrylate = 85:15.

One hundred of the resulting particles were randomly sampled and their shapes examined with the SEM. The particles were found to be elliptical, needle-shaped or rod-shaped polymer particles. The $L_{AV}$ was 16.8 $\mu$m, and the $P_{AV}$ was 1.9.

The particles were checked for extraneous matter, but substantially no deformed matter such as agglomerates and mutually sticking particles was observed.

A portion of the resulting particles was measured with a Fourier transform infrared spectrophotometer. The peak at a wave number of 910 (cm$^{-1}$) attributable to epoxy groups was confirmed to be smaller than before synthesis.

[Comparative Example 1]

Aside from not carrying out pH adjustment, a methyl methacrylate-glycidyl methacrylate copolymer particle dispersion was obtained in the same way as in Working Example 1. The pH of the synthesis solution before the start of heating was 7, the pH 2 hours after the starting of heating was 7, and the pH at the time of reaction completion was 7.
The resulting particle dispersion was transferred to a separate 3,000 mL flask and the synthesis vessel (the flask and the stirring element) was checked for deposits; masses of agglomerate from precipitated polymer were observed to some degree on the inner periphery of the flask and on the stirring element.
Next, using a known suction filtration apparatus, the separately transferred particle dispersion was repeatedly (5 times) washed with methanol and filtered, and then vacuum dried, giving Polymer Particle B1. The makeup (molar ratio) of the respective recurring units in Polymer Particle B1 was methyl methacrylate : glycidyl methacrylate = 89:11.
One hundred of the resulting particles were randomly sampled and their shapes examined with the SEM. Most of the particles were elliptical, needle-shaped or rod-shaped polymer particles. The $L_{AV}$ was 14.5 $\mu$m, and the $P_{AV}$ was 5.5.
A portion of the resulting particles was measured with a Fourier transform infrared spectrophotometer. The peak at a wave number of 910 (cm$^{-1}$) attributable to epoxy groups was unchanged compared with before synthesis.

[Comparative Example 2]

A synthesis solution was prepared by dissolving the compounds indicated below in the respective phases, mixing together the aqueous phase and the oil phase, and charging the mixture into a 2,000 mL flask. The synthesis solution was then stirred for one hour, after which, under a stream of nitrogen, the solution was set to an oil bath temperature of 80°C and heated and stirred for 12 hours, giving a methyl methacrylate-glycidyl methacrylate copolymer particle dispersion. The pH of the synthesis solution before the start of heating was 7, the pH 2 hours after the start of heating was 6, and the pH at the time of reaction completion was 6.

Aqueous Phase

| | |
|---|---|
| Water | 1,280.0 g |
| Polyvinyl pyrrolidone (K-15) | 8.0 g |
| Ammonium persulfate | 4.8 g |

Oil Phase

| | |
|---|---|
| Toluene | 80.0 g |
| Polystyrene (Mw, 45,000) | 16.0 g |
| Methyl methacrylate | 128.0 g |
| Glycidyl methacrylate | 32.0 g |

(Polystyrene: Polystyrene from Aldrich Co.; average Mw, ca. 45,000)
The resulting particle dispersion was transferred to a separate 3,000 mL flask and the synthesis vessel (the flask and the stirring element) was checked for deposits; masses of agglomerate from precipitated polymer were observed to some degree on the inner periphery of flask and on the stirring element.
Next, using a known suction filtration apparatus, the separately transferred particle dispersion was repeatedly (5 times) washed with methanol, filtered and classified, and then vacuum dried, giving Polymer Particle B2. The makeup (molar ratio) of the respective recurring units in Polymer Particle B2 was methyl methacrylate : glycidyl methacrylate = 85:15.
One hundred of the resulting particles were randomly sampled and their shapes examined with the SEM. Most of the particles were elliptical, needle-shaped or rod-shaped polymer particles. The $L_{AV}$ was 21.6 $\mu$m, and the $P_{AV}$ was 5.9.
A portion of the resulting particles was measured with a Fourier transform infrared spectrophotometer. The peak at a wave number of 910 (cm$^{-1}$) attributable to epoxy groups was unchanged compared with before synthesis.

[Comparative Example 3]

A suspension was prepared by charging the compounds shown below all at once into a 2,000 mL flask, and stirring at 1,000 rpm with a dispersion mixer dispersing element. Next, the suspension was set to an oil bath temperature of 80°C and heated and stirred for 8 hours under a stream of nitrogen, giving a methyl methacrylate-glycidyl methacrylate copolymer particle dispersion.

| Water | 1,325.0 g |
| Methyl methacrylate | 134.4 g |
| Glycidyl methacrylate | 33.6 g |
| Lauryl peroxide | 8.3 g |
| Polyvinyl pyrrolidone (K-30) | 17.0 g |

The resulting particle dispersion was transferred to a separate 3,000 mL flask and the flask and stirring element were checked for deposits; masses of agglomerate from precipitated polymer were observed to some degree on the inner periphery of flask and on the stirring element.

Next, centrifugal separation from the separately transferred particle dispersion was repeated five times, and classifying and washing operations were carried out, giving spherical polymer particles B3 of polymethyl methacrylate alone having an average particle size of 5 $\mu$m. The makeup (molar ratio) of the respective recurring units in Polymer Particle B3 was methyl methacrylate : glycidyl methacrylate = 85:15.

The shapes of the resulting particles were examined with the SEM. The particles were found to be spherical polymer particles having a volume mean particle size of 5 $\mu$m.

A portion of the resulting particles was measured with a Fourier transform infrared spectrophotometer. The peak at a wave number of 910 (cm$^{-1}$) attributable to epoxy groups was unchanged compared with before synthesis.

The MV, L$_{AV}$, D$_{AV}$, P$_{AV}$, particle ingredients and shape for the particles obtained in Working Examples 1 to 6 and Comparative Examples 1 to 3 are shown below in Table 1.

[Table 1]

| | | Polymer particle | MV ($\mu$m) | L$_{AV}$ ($\mu$m) | D$_{AV}$ ($\mu$m) | P$_{AV}$ | Main shape | Constituents |
|---|---|---|---|---|---|---|---|---|
| Working Example | 1 | A1 | 7.8 | 18.2 | 2.8 | 6.5 | elliptical/ needle-shaped | methyl methacrylate glycidyl methacrylate |
| | 2 | A2 | 5.6 | 16.1 | 2.1 | 8.1 | elliptical/ needle-shaped | methyl methacrylate glycidyl methacrylate |
| | 3 | A3 | 8.7 | 31.6 | 3.1 | 10.5 | elliptical/ needle-shaped | styrene methacrylic acid glycidyl methacrylate |
| | 4 | A4 | 3.5 | 12.4 | 1.7 | 8.7 | elliptical/ needle-shaped | styrene glycidyl methacrylate |
| | 5 | A5 | 9.1 | 24.8 | 3.5 | 7.2 | elliptical/ needle-shaped | styrene methacrylamide glycidyl methacrylate |
| | 6 | A6 | 10.1 | 16.8 | 8.2 | 1.9 | elliptical | methyl methacrylate glycidyl methacrylate |
| Comparative Example | 1 | B1 | 7.6 | 14.5 | 2.4 | 5.5 | elliptical/ needle-shaped | methyl methacrylate glycidyl methacrylate |
| | 2 | B2 | 10.2 | 21.6 | 4.1 | 5.9 | elliptical/ needle-shaped | methyl methacrylate glycidyl methacrylate |

(continued)

| | | Polymer particle | MV (μm) | $L_{AV}$ (μm) | $D_{AV}$ (μm) | $P_{AV}$ | Main shape | Constituents |
|---|---|---|---|---|---|---|---|---|
| | 3 | B3 | 5.0 | - | - | 1.0 | spherical | methyl methacrylate glycidyl methacrylate |

The SB, SD, water absorption and oil absorption for the particles obtained in Working Examples 1 to 6 and Comparative Examples 1 to 3 are shown below in Table 2.

[Table 2]

| | | Polymer particle | $(m^2/)g$ | SD $(m^2/g)$ | SB/SD | Water absorption (mL/100 g) | Oil absorption (mL/100 g) |
|---|---|---|---|---|---|---|---|
| Working Example | 1 | A1 | 2.3585 | 0.641 | 3.68 | 89.2 | 129.8 |
| | 2 | A2 | 3.3217 | 0.893 | 3.72 | 81.4 | 119.2 |
| | 3 | A3 | 2.1521 | 0.575 | 3.74 | 101.4 | 142.5 |
| | 4 | A4 | 3.5633 | 1.429 | 2.49 | 74.1 | 101.9 |
| | 5 | A5 | 2.4389 | 0.549 | 4.44 | 98.6 | 133.2 |
| | 6 | A6 | 2.0564 | 0.495 | 4.15 | 78.2 | 100.1 |
| Comparative Example | 1 | B1 | 3.2648 | 0.658 | 4.96 | 106.3 | 140.1 |
| | 2 | B2 | 2.6568 | 0.490 | 5.42 | 119.2 | 156.2 |
| | 3 | B3 | 0.7435 | 1.000 | 0.74 | 48.0 | 53.9 |

[2] Chemical Resistance Test

One gram of the respective particles obtained in Working Examples 1 to 6 and Comparative Examples 1 to 3 and 99 g of the solvent indicated in the table below were placed in a 300 mL flask (particle concentration, 1 wt%) and stirred for 30 minutes at 27°C, following which the dispersed state of the particles was visually checked.

Using the particles obtained by subsequently carrying out filtration with a suction filter, washing away the filtered matter with reused solvent and water, and drying the solids, measurement of the percent loss in weight and verification of the particle shape by SEM were carried out.

$$\text{Loss in weight (\%)} =$$

$$(1 - (\text{particle weight after test})/(\text{particle weight before test})) \times 100$$

Table 3 shows the results obtained by checking the particle shapes visually and by SEM. Table 4 shows the percent loss in weight.

[Table 3]

| Solvent | | Water | | Ethanol | | Ethyl acetate | | Dimethylformamide | | Methyl ethyl ketone | | Dipropylene glycol | | Acetone | | Toluene | |
|---|---|---|---|---|---|---|---|---|---|---|---|---|---|---|---|---|---|
| | | Visual | SEM | Visual | SEM | Visual | SEM | Visual | SEM | Visual | SEM | Visual | SEM | Visual | SEM | Visual | SEM |
| Working Example | 1 | ○ | 1 | ○ | 1 | ○ | 1 | ○ | 1 | ○ | 1 | ○ | 1 | ○ | 1 | ○ | 1 |
| | 2 | ○ | 1 | ○ | 1 | ○ | 1 | ○ | 1 | ○ | 1 | ○ | 1 | ○ | 1 | ○ | 1 |
| | 3 | ○ | 1 | ○ | 1 | ○ | 1 | ○ | 1 | ○ | 1 | ○ | 1 | ○ | 1 | ○ | 1 |
| | 4 | ○ | 1 | ○ | 1 | ○ | 1 | ○ | 1 | ○ | 1 | ○ | 1 | ○ | 1 | ○ | 1 |
| | 5 | ○ | 1 | ○ | 1 | ○ | 1 | ○ | 1 | ○ | 1 | ○ | 1 | ○ | 1 | ○ | 1 |
| | 6 | ○ | 1 | ○ | 1 | ○ | 1 | ○ | 1 | ○ | 1 | ○ | 1 | ○ | 1 | ○ | 1 |
| Comparative Example | 1 | ○ | 1 | Δ | 2 | X | 3 | X | 3 | X | 3 | Δ | 2 | X | 3 | X | 3 |
| | 2 | ○ | 1 | Δ | 2 | X | 3 | X | 3 | X | 3 | Δ | 2 | X | 3 | X | 3 |
| | 3 | ○ | 1 | Δ | 2 | X | 3 | X | 3 | X | 3 | Δ | 2 | X | 3 | X | 3 |

○: Dispersed
Δ: Partially dispersed
X: Dissolved
1: Shapes are those of prepared particles
2: Some deformation
3: Shapes of prepared particles absent

[Table 4]

| Solvent | | Weight loss ratio (%) | | | | | | | |
|---|---|---|---|---|---|---|---|---|---|
| | | Water | Ethanol | Ethyl acetate | Dimethylformamide | Methyl ethyl ketone | Dipropylene glycol | Acetone | Toluene |
| Working Example | 1 | 0.0 | 0.1 | 0.1 | 0.1 | 0.2 | 0.0 | 0.1 | 0.1 |
| | 2 | 0.0 | 0.1 | 0.2 | 0.2 | 0.2 | 0.0 | 0.2 | 0.1 |
| | 3 | 0.0 | 0.2 | 0.4 | 0.3 | 0.3 | 0.2 | 0.2 | 0.3 |
| | 4 | 0.0 | 0.2 | 0.3 | 0.4 | 0.2 | 0.1 | 0.3 | 0.4 |
| | 5 | 0.0 | 0.4 | 1.0 | 0.6 | 0.6 | 0.3 | 0.7 | 1.1 |
| | 6 | 0.0 | 0.0 | 0.1 | 0.1 | 0.1 | 0.0 | 0.0 | 0.1 |
| Comparative Example | 1 | 0.0 | 4.4 | N | N | N | 16.3 | N | N |
| | 2 | 0.0 | 3.1 | N | N | N | 11.7 | N | N |
| | 3 | 0.0 | 2.7 | N | N | N | 13.1 | N | N |
| N: Because of dissolution, no particles were obtained after drying. | | | | | | | | | |

These results demonstrate that the elliptical, needle-shaped or rod-shaped crosslinked polymer particles of the invention have an excellent chemical resistance.

[3] Heat-Resistance Test

An amount of 0.5 g of the respective particles obtained in Working Examples 1 to 6 and Comparative Examples 1 to 3 was placed in an aluminum Petri dish and heated for 2 hours in a drying oven at the temperature shown in Table 5, following which the particles were visually checked for melting. In addition, the particle shapes were checked with the SEM. The evaluation results are presented in Table 5.

[Table 5]

| Temperature | | 80°C | | 100°C | | 120°C | | 150°C | | 200°C | | 250°C | |
|---|---|---|---|---|---|---|---|---|---|---|---|---|---|---|
| | | Visual | SEM | Visual | SEM | Visual | SEM | Visual | SEM | Visual | SEM | Visual | SEM |
| Example | 1 | ○ | 1 | ○ | 1 | ○ | 1 | ○ | 1 | ○ | 1 | ○ | 1 |
| | 2 | ○ | 1 | ○ | 1 | ○ | 1 | ○ | 1 | ○ | 1 | ○ | 1 |
| | 3 | ○ | 1 | ○ | 1 | ○ | 1 | ○ | 1 | ○ | 1 | ○ | 1 |
| | 4 | ○ | 1 | ○ | 1 | ○ | 1 | ○ | 1 | ○ | 1 | ○ | 1 |
| | 5 | ○ | 1 | ○ | 1 | ○ | 1 | ○ | 1 | ○ | 1 | Δ | 2 |
| | 6 | ○ | 1 | ○ | 1 | ○ | 1 | ○ | 1 | ○ | 1 | ○ | 1 |
| Comparative Example | 1 | ○ | 1 | Δ | 2 | Δ | 2 | X | 3 | X | 3 | X | 3 |
| | 2 | ○ | 1 | Δ | 2 | Δ | 2 | X | 3 | X | 3 | X | 3 |
| | 3 | ○ | 1 | Δ | 2 | Δ | 2 | X | 3 | X | 3 | X | 3 |

O: No major change
Δ: Partially melted
X: Melted
1: Shapes are those of prepared particles
2: Shape of some particles retained
3: Shapes of prepared particles absent

These results demonstrate that the elliptical, needle-shaped or rod-shaped crosslinked polymer particles of the invention have excellent heat resistance.

[4] Hot Chemical Resistance Test

One gram of the respective particles obtained in Working Examples 1 to 6 and Comparative Examples 1 to 3 and 99 g of the solvent indicated in the table below were placed in a 300 mL flask (particle concentration, 1 wt%) and stirred for 30 minutes at 70°C, following which the dispersed state of the particles was visually checked.

Using the particles obtained by subsequently carrying out filtration with a suction filter, washing away the filtered matter with reused solvent and water, and drying the solids, measurement of the percent loss in weight and verification of the particle shapes by SEM were carried out.

$$\text{Loss in weight (\%)} =$$

$$(1 - (\text{particle weight after test})/(\text{particle weight before test})) \times 100$$

Table 6 shows the results obtained by checking the particle shapes visually and by SEM. Table 7 shows the percent loss in weight.

[Table 6]

| Solvent | | Water | | Ethanol | | Ethyl acetate | | Dimethylformamide | | Methyl ethyl ketone | | Toluene | | Dipropylene glycol | |
|---|---|---|---|---|---|---|---|---|---|---|---|---|---|---|---|
| | | Visual | SEM | Visual | SEM | Visual | SEM | Visual | SEM | Visual | SEM | Visual | SEM | Visual | SEM |
| Working Example | 1 | ○ | 1 | ○ | 1 | ○ | 1 | ○ | 1 | ○ | 1 | ○ | 1 | ○ | 1 |
| | 2 | ○ | 1 | ○ | 1 | ○ | 1 | ○ | 1 | ○ | 1 | ○ | 1 | ○ | 1 |
| | 3 | ○ | 1 | ○ | 1 | ○ | 1 | ○ | 1 | ○ | 1 | ○ | 1 | ○ | 1 |
| | 4 | ○ | 1 | ○ | 1 | ○ | 1 | ○ | 1 | ○ | 1 | ○ | 1 | ○ | 1 |
| | 5 | ○ | 1 | ○ | 1 | ○ | 1 | ○ | 1 | ○ | 1 | ○ | 1 | ○ | 1 |
| | 6 | ○ | 1 | ○ | 1 | ○ | 1 | ○ | 1 | ○ | 1 | ○ | 1 | ○ | 1 |
| Comparative Example | 1 | ○ | 1 | Δ | 2 | X | 3 | X | 3 | X | 3 | X | 3 | X | 3 |
| | 2 | ○ | 1 | Δ | 2 | X | 3 | X | 3 | X | 3 | X | 3 | X | 3 |
| | 3 | ○ | 1 | Δ | 2 | X | 3 | X | 3 | X | 3 | X | 3 | X | 3 |

O: Dispersed
Δ: Partially dispersed
X: Dissolved
1: Shapes are those of prepared particles
2: Some deformation
3: Shapes of prepared particles absent

[Table 7]

| Solvent | | Weight loss ratio (%) | | | | | | |
|---|---|---|---|---|---|---|---|---|
| | | Water | Ethanol | Ethyl acetate | Dimethylformamide | Methylethylketone | Toluene | Dipropylene glycol |
| Working Example | 1 | 0.0 | 0.1 | 0.1 | 0.2 | 0.3 | 0.4 | 0.1 |
| | 2 | 0.0 | 0.1 | 0.4 | 0.3 | 0.4 | 0.3 | 0.2 |
| | 3 | 0.1 | 0.4 | 0.6 | 0.7 | 0.8 | 0.8 | 0.4 |
| | 4 | 0.0 | 0.4 | 0.5 | 0.8 | 0.6 | 0.9 | 0.2 |
| | 5 | 0.0 | 0.8 | 2.0 | 1.3 | 1.2 | 1.8 | 1.4 |
| | 6 | 0.0 | 0.1 | 0.1 | 0.2 | 0.2 | 0.3 | 0.1 |
| Comparative Example | 1 | 1.1 | 19.2 | N | N | N | N | N |
| | 2 | 0.8 | 16.4 | N | N | N | N | N |
| | 3 | 0.4 | 17.1 | N | N | N | N | N |

N: Because of dissolution, no particles were obtained after drying.

These results demonstrate that the elliptical, needle-shaped or rod-shaped crosslinked polymer particles of the invention have an excellent resistance to hot chemicals.

[5] Preparation and Evaluation of Optical Measurement Sheet

Polymer Particle A1 (Working Example 1), Polymer Particle B1 (Comparative Example 1) or Polymer Particle B3 (Comparative Example 3), all having similar volumetric particle sizes, were added as respective 15 wt% diffusing agents to an acrylic molding resin (Delpet 80NB, from Asahi Kasei Chemicals Corporation). After master pelletizing and drying steps, melt kneading was carried out at 230°C using a twin-screw extruder, and test sheets having a film thickness of 2 mm were prepared using a T-die. The resulting sheets were designated as Optical Sheets 1 to 3.

[Working Example 7, Comparative Examples 4 and 5]

The appearances of the resulting Optical Sheets 1 to 3 were visually examined, and the particle shapes were checked with the SEM. The results are presented in Table 8.

[Table 8]

| | Optical sheet | Polymer particles (wt%) | | | Binder resin | Visual | SEM |
|---|---|---|---|---|---|---|---|
| | | A1 | B1 | B3 | | | |
| Working Example 7 | 1 | 15 | - | - | Polymethyl methacrylate | ○ | 1 |
| Comparative Example 4 | 2 | - | 15 | - | Polymethyl methacrylate | X | 3 |
| Comparative Example 5 | 3 | - | - | 15 | Polymethyl methacrylate | X | 3 |

Visual:

O: Cloudy sheet

X: Transparent or translucent sheet

SEM:

1: Particle shape is retained

2: Deformed or partially melted

3: Substantially melted

[Working Example 8, Comparative Examples 6 and 7]

Measurements of total light transmittance and haze for Optical Sheets 1 to 3 were carried out using the NDH-5000 Haze Meter (Nippon Denshoku Industries Co., Ltd.). The results are shown in Table 9.

[Table 9]

| | Optical sheet | Total light transmittance (%) | Haze (%) | Particle shape |
|---|---|---|---|---|
| Working Example 8 | 1 | 46.3 | 89.6 | ellipticafneedle-shaped |
| Comparative Example 6 | 2 | 80.2 | 11.4 | substantially melted |
| Comparative Example 7 | 3 | 87.5 | 4.0 | substantially melted |

These results demonstrate that light-diffusing sheets having heat resistance can be obtained using the elliptical, needle-shaped or rod-shaped polymer particles of the invention.

[6] Evaluation of Reflected Light Scattering Ability

[Working Example 9, Comparative Example 8]

Test sheets were prepared by uniformly applying 0.24 mg/cm$^2$ of Polymer Particle A2 (Working Example 9) or Polymer Particle B3 (Comparative Example 3) having similar volumetric particle sizes onto black synthetic leather (5 cm $\times$ 8 cm) while patting with a cosmetic powder puff. Next, using an automated goniophotometer (GP-200, from Murakami Color Research Laboratory Co., Ltd.), a fixed amount of light was irradiated onto the test sheet at an incident angle of 45° and the light scattering distribution of the reflected light was measured. The results are shown in FIG. 2.

From the results in FIG. 2, the elliptical, needle-shaped or rod-shaped crosslinked polymer particles of the invention have an excellent reflected light scattering ability, and so it was confirmed that the optical characteristics distinctive to elliptical particles can be retained.

[7] Evaluation of UV Transmitted Light

[Working Example 10, Comparative Example 11]

A composition was prepared by mixing Polymer Particle A2 (Working Example 10) or Polymer Particle B3 (Comparative Example 9), both of which had similar volumetric particle sizes, with a binder resin (PVA resin from Kuraray Co., Ltd.) and purified water in the proportions shown in Table 10 below, following which the composition was coated onto one side of a 100 μm thick PET film (E-5000, from Toyobo Co., Ltd.) using a commercial bar coater. After coating, a drying oven was set to 60°C and forced hot-air drying was carried out for 20 minutes, thereby producing Optical Sheets 4 and 5. The thickness of the applied layer on Optical Sheets 4 and 5 was 40 μm.

[Table 10]

| | Optical sheet | Polymer particles (g) | | Binder resin (g) | Purified water (g) |
|---|---|---|---|---|---|
| | | A2 | B3 | | |
| Working Example 10 | 4 | 15.0 | - | 35.0 | 75.0 |
| Comparative Example 9 | 5 | - | 15.0 | 35.0 | 75.0 |

UV transmission spectroscopy at wavelengths of 320 nm, 360 nm and 400 nm was carried out on Optical Sheets 4 and 5 using a UV-visible spectrophotometer (UV-2450, from JASCO Corporation). The results are shown in Table 11.

[Table 11]

| | Optical sheet | Transmittance (%) | | | Particle shape |
|---|---|---|---|---|---|
| | | 320 nm | 360 nm | 400 nm | |
| Working Example 10 | 4 | 3.6 | 5.2 | 7.3 | elliptical/needle-shaped |
| Comparative Example 9 | 5 | 13.1 | 31.4 | 34.3 | spherical |

The results of UV transmission spectroscopy demonstrate that the elliptical, needle-shaped or rod-shaped crosslinked polymer particles of the invention, given the decrease in transmitted light in the UV region (especially, UV-A), clearly have a large UV scattering effect, and that optical characteristics distinctive to an elliptical shape are maintained.

The evaluation results for reflective scattering and UV transmitted light demonstrate that, because the elliptical, needle-shaped or rod-shaped polymer particles of the invention have high scattering effects in the visible and UV light regions, the hiding power is also high.

[8] Sensory Tests and Evaluation of Adhesion

[Working Example 11, Comparative Example 10]

Polymer Particle A2 (Working Example 11) and Polymer Particle B3 (Comparative Example 10), both having similar volumetric particle sizes, were evaluated by the methods shown below. The results are shown in Table 12.

<Evaluated Qualities>

| | |
|---|---|
| Feel: | The tactile feel of each type of particle when spread over the skin was evaluated. |
| Slip characteristics: | The slip characteristics were evaluated by placing 1 g of each type of particle on black synthetic leather, and measuring the length when spread with a finger. |
| Particle adhesion: | One gram of each type of particle was placed on black synthetic leather and uniformly spread with a powder puff, following which the leather was struck three times and the amount of particles remaining was examined with a digital microscope (VHX200, from Keyence Corporation). |

<Evaluation Criteria>

Feel:

O: Tactile sensation is apparent, with application to skin being pleasant
Δ: Tactile sensation is apparent, but ordinary
X: Tactile sensation is apparent, but unpleasant

Slip characteristics:

O: Particles spread well
Δ: Ordinary
X: Particles do not spread

Particle adhesion:

O: Adhered state is substantially maintained
Δ: Local peeling observed here and there
X: Most of the applied particles peeled and fell off

[Table 12]

| | Particles | Feel | Slip | Particle adhesion | Major shape |
|---|---|---|---|---|---|
| Working Example 11 | A2 | O | O | O | elliptical/needle-shaped |
| Comparative Example 10 | B3 | Δ | O | X | spherical |

It was confirmed from these results that, because the feel, slip and particle adhesion properties in Working Example 11 are excellent, characteristics distinctive to an elliptical shape are exhibited.
The above results demonstrate that the elliptical, needle-shaped or rod-shaped crosslinked polymer particles obtained according to this invention, along with having heat resistance and chemical resistance, also fully retain the properties inherent to the elliptical shape. Hence, it is possible to expand their use to applications requiring heat resistance, chemical resistance and hot chemical resistance, and so they are expected to be capable of effective use in applications such as paints, inks, molded or formed articles, cosmetics, optical materials such as light diffusing sheets, and thermally cavitated products having pores.

**Claims**

1. An elliptical, needle-shaped or rod-shaped crosslinked polymer particle including, as 5 to 100 mol% of all recurring units, recurring units derived from an unsaturated monomer having a functional group selected from epoxy, carboxyl, amide, hydroxyl, amino and thiol groups, wherein

(1) a two-dimensional projection obtained by irradiating the particle with light from a direction orthogonal to a long axis of the particle has a length L with an average value $L_{AV}$ of from 0.1 to 80 $\mu$m,

(2) a two-dimensional projection obtained by irradiating the particle with light from a direction orthogonal to a long axis of the particle has a breadth D with an average value $D_{AV}$ of from 0.05 to 40 $\mu$m, and

(3) the aspect ratio L/D calculated from the length L and breadth D has an average value $P_{AV}$ of from 1.5 to 30.

2. The elliptical, needle-shaped or rod-shaped crosslinked polymer particle of claim 1, wherein the functional group is an epoxy, carboxyl, amino or amide group.

3. The elliptical, needle-shaped or rod-shaped crosslinked polymer particle of claim 1 or 2, wherein the crosslinked polymer further includes recurring units derived from at least one type of unsaturated monomer selected from styrenic monomers, (meth)acrylic ester monomers and vinyl carboxylate monomers.

4. The elliptical, needle-shaped or rod-shaped crosslinked polymer particle of any one of claims 1 to 3, wherein the ratio SB/SD between the actual specific surface area SB of the particle and the theoretical specific surface area SD of a spherical particle calculated from the volume mean particle size of the elliptical, needle-shaped or rod-shaped crosslinked polymer particle satisfies the condition SB/SD $\geq$ 1.2.

5. The elliptical, needle-shaped or rod-shaped crosslinked polymer particle of any one of claims 1 to 4 wherein, when the crosslinked polymer particles are added to at least one solvent selected from the group consisting of ethanol, toluene, ethyl acetate, methyl ethyl ketone, dimethylformamide and dipropylene glycol and stirred for 30 minutes at 27°C, the percent loss in weight is not more than 10 wt%.

6. The elliptical, needle-shaped or rod-shaped crosslinked polymer particle of any one of claims 1 to 5 wherein, when the crosslinked polymer particles are added to at least one solvent selected from the group consisting of ethanol, toluene, ethyl acetate, methyl ethyl ketone, dimethylformamide and dipropylene glycol and stirred for 30 minutes at 70°C, the percent loss in weight is not more than 10 wt%.

7. A resin composition obtained using the elliptical, needle-shaped or rod-shaped crosslinked polymer particle of any one of claims 1 to 6.

8. A light-diffusing sheet obtained using the elliptical, needle-shaped or rod-shaped crosslinked polymer particle of any one of claims 1 to 6.

9. A paint composition obtained using the elliptical, needle-shaped or rod-shaped crosslinked polymer particle of any one of claims 1 to 6.

10. An ink composition obtained using the elliptical, needle-shaped or rod-shaped crosslinked polymer particle of any one of claims 1 to 6.

11. A cosmetic preparation obtained using the elliptical, needle-shaped or rod-shaped crosslinked polymer particle of any one of claims 1 to 6.

12. A material for the electrical or electronics industry obtained using the elliptical, needle-shaped or rod-shaped crosslinked polymer particle of any one of claims 1 to 6.

13. An adhesive obtained using the elliptical, needle-shaped or rod-shaped crosslinked polymer particle of any one of claims 1 to 6.

14. A thermally cavitated product having pores obtained using the elliptical, needle-shaped or rod-shaped crosslinked polymer particle of any one of claims 1 to 6.

15. A diagnostic agent for medical use obtained using the elliptical, needle-shaped or rod-shaped crosslinked polymer particle of any one of claims 1 to 6.

16. A method for producing the elliptical, needle-shaped or rod-shaped crosslinked polymer particle of any one of claims 1 to 6, comprising the step of carrying out solution polymerization by heating a synthesis solution containing a mixed solvent of water, a hydrophilic organic solvent and a hydrophobic organic solvent, a high-molecular-weight stabilizer, a polymerization initiator and the unsaturated monomer; and, at least following the start of heating, adjusting the pH of the synthesis solution to 5 or less or to 9 or more.

17. The method for producing the elliptical, needle-shaped or rod-shaped crosslinked polymer particle of claim 16, wherein the mixing ratio of the water, hydrophilic organic solvent and hydrophobic organic solvent, expressed as a weight ratio, is from 99:0.5:0.5 to 25:55:20.

18. The method for producing the elliptical, needle-shaped or rod-shaped crosslinked polymer particle of claim 16 or 17, wherein the hydrophobic organic solvent is an organic compound having a molecular weight of at least 200.

# FIG.1

# FIG.2

—— EXAMPLE 9
- - - COMPARATIVE EXAMPLE 8

# INTERNATIONAL SEARCH REPORT

| | International application No. |
|---|---|
| | PCT/JP2016/063543 |

| A. CLASSIFICATION OF SUBJECT MATTER |
|---|
| C08F2/04(2006.01)i |

According to International Patent Classification (IPC) or to both national classification and IPC

| B. FIELDS SEARCHED |
|---|
| Minimum documentation searched (classification system followed by classification symbols) |
| C08F2/00-2/60, C08C19/00-19/44, C08F6/00-246/00, C08F301/00 |

Documentation searched other than minimum documentation to the extent that such documents are included in the fields searched
Jitsuyo Shinan Koho 1922–1996 Jitsuyo Shinan Toroku Koho 1996–2016
Kokai Jitsuyo Shinan Koho 1971–2016 Toroku Jitsuyo Shinan Koho 1994–2016

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| A | JP 2009-235355 A (Nisshinbo Holdings Inc.), 15 October 2009 (15.10.2009), entire text (Family: none) | 1-18 |
| A | JP 2009-237507 A (Nisshinbo Holdings Inc.), 15 October 2009 (15.10.2009), entire text (Family: none) | 1-18 |
| A | JP 2010-518208 A (BASF SE), 27 May 2010 (27.05.2010), entire text & JP 5328675 B2 & US 2010/0062932 A1 entire text & WO 2008/095892 A1 & EP 2115013 A1 & CN 101605818 A | 1-18 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 July 2016 (12.07.16) | 19 July 2016 (19.07.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**INTERNATIONAL SEARCH REPORT**

| International application No. |
| --- |
| PCT/JP2016/063543 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2008-111132 A (Soken Chemical & Engineering Co., Ltd.), 15 May 2008 (15.05.2008), entire text (Family: none) | 1-18 |
| A | JP 2007-326904 A (Fujifilm Corp.), 20 December 2007 (20.12.2007), entire text & US 2008/0038669 A1 entire text | 1-18 |
| P,A | JP 2016-017048 A (Nisshinbo Holdings Inc.), 01 February 2016 (01.02.2016), entire text (Family: none) | 1-18 |
| P,A | JP 2015-093973 A (Nisshinbo Holdings Inc.), 18 May 2015 (18.05.2015), entire text & WO 2015/072443 A1 entire text | 1-18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009235353 A

- JP 2009235355 A